# EUROPEAN PATENT APPLICATION

(11) **EP 4 410 953 A1**
(43) Date of publication of application: **07.08.2024**
(21) Application number: 22876959.2
(22) Date of filing: 30.09.2022
(51) Int. Cl.: C12N 1/20, A61K 35/747, A61K 35/745, A61K 35/744, A23L 33/135

(54) **NOVEL PROBIOTICS AND USE THEREOF**

(30) Priority: 01.10.2021 KR 20210130539
(71) Applicant: Pblbiolab Co., Ltd., Seoul 02823 (KR); NVP Healthcare Co., Ltd., Suwon-si, Gyeonggi-do 16209 (KR)
(72) Inventor: KIM, Dong-Hyun, Seoul 02823 (KR)
(74) Representative: Cabinet Becker et Associés
(86) International application number: PCT/KR2022/014798
(87) International publication number: WO 2023/055188

(57) **Abstract**

The present invention relates to Lactobacillus rhamnosus, Bifidobacterium longum, and Lactococcus lactis, which are novel lactic acid bacteria. The strains according to the present invention have high utility as a medicine or food and, in particular, can be used for preventing, treating, or relieving inflammatory diseases and neurological diseases, due to having an excellent antioxidant effect, inflammation-relieving effect, nerve-improving effect, cognition-improving effect, intestinal microbiota imbalance-improving effect, etc. In addition, the present invention can be used for regulating and enhancing immunity by increasing the activity of the immune system through immune function-improving and immunity-enhancing effects.

## Description

### [Technical Field]

The present disclosure relates to novel probiotics, specifically, *Lactobacillus rhamnosus, Bifidobacterium longum,* and *Lactococcus lactis.*

### [Background Art]

Probiotics are bioactive agents that are distributed as dominant bacteria in the human intestine where various microorganisms exist and promote the growth of good bacteria in the body, and live in symbiosis with the human digestive system and play in a role in breaking down fiber and complex proteins into important nutritional components. They also inhibit the growth of harmful bacteria such as *E. coli* and *Clostridium difficile,* improve diarrhea and constipation, synthesize vitamins, and lower blood cholesterol. Among these probiotics, lactic acid bacteria are bacteria that ferment sugars to obtain energy and produce large amounts of lactic acid. They are widely distributed in the natural world, including in agricultural products, food, and the bodies of humans and animals. They are widely used in fermentation of cheese, fermented milk, kimchi, and bread. In general, it is known that the consumption of lactic acid bacteria not only suppresses harmful bacteria among intestinal microorganisms, but also increases good bacteria, contributing to the digestion, absorption, and decomposition of food. Therefore, consuming lactic acid bacteria has been reported to offer various effects, including lowering blood cholesterol, enhancing immunity, suppressing endogenous infections, improving liver cirrhosis, and exhibiting anticancer effects. In addition, research has recently been conducted on increasing the efficacy of natural products fermented with lactic acid bacteria.

Microbiome is a combination of the words "microbe" and "biome". Some researchers sometimes refer to the microbiome as the 'second genome (genetic information)'. Each person possesses a unique microbiome, much like the individuality found in fingerprints. This difference determines the incidence of various diseases from allergies, atopy, and obesity to enteritis and heart disease. Meanwhile, since 95% or more of the microbiome resides within the intestines, intestinal bacteria are receiving greater attention.

When the healthy ratio of good to bad bacteria in gut intestinal flora is disrupted, immunity is greatly compromised. This is because good bacteria in the gut induce immune cell activation. In addition, as the proportion of bad bacteria increases, the defensive barrier function of the intestines weakens, leading to damage in the intestinal mucosa. As a result, pathogens and toxins present in the intestinal tract may enter the bloodstream, causing infectious diseases or autoimmune diseases. There are also assertions suggesting that gut bacteria increase the risk of dementia.

Accordingly, increasing the level of target good bacteria by changing the gut microflora is essential for improving complex health functions. To achieve this, it is imperative for deriving an appropriate strain from countless strains, but it is very difficult to find these specific strains.

Against this backdrop, the present inventors have identified novel strains that may have preventive, improving, and therapeutic effects in inflammatory diseases and neurological disorders and completed the present disclosure.

### [Disclosure]

### [Technical Problem]

An object of the present disclosure is to provide novel probiotics, specifically, *Lactobacillus rhamnosus, Bifidobacterium longum,* and *Lactococcus lactis.*

Another object of the present disclosure is to provide the use of the novel probiotics.

### [Technical Solution]

In one general aspect, the present disclosure provides *Lactobacillus rhamnosus* NK210 (Depository Institution: Korean Culture Center of Microorganisms, Deposit Date: September 15, 2021, Accession Number: KCCM13049P).

*Lactobacillus rhamnosus* NK210 of the present disclosure is characterized as a novel probiotic of *Lactobacillus rhamnosus* isolated and identified from human feces.

The 16S rDNA sequence for identification and classification of *Lactobacillus rhamnosus* NK210 of the present disclosure is the same as SEQ ID NO: 1 attached to the present specification. Therefore, *Lactobacillus rhamnosus* NK210 of the present disclosure may comprise 16S rDNA of SEQ ID NO: 1.

Analysis of the 16S rDNA sequence of SEQ ID NO: 1 showed 99% homology with known *Lactobacillus rhamnosus* strains, indicating the highest molecular phylogenetic relationship with *Lactobacillus rhamnosus.* Therefore, the lactic acid bacterium was identified as *Lactobacillus rhamnosus,* named *Lactobacillus rhamnosus* NK210, and deposited at the Korean Culture Center of Microorganisms on September 15, 2021 (Accession Number: KCCM13049P).

*Lactobacillus rhamnosus* NK210 of the present disclosure is a Gram-positive bacterium, and its cell morphology is bacillus. More specific physiological characteristics of *Lactobacillus rhamnosus* NK210 may be analyzed according to conventional methods in the art, and the results are shown in Table 3 below. Specifically, *Lactobacillus rhamnosus* NK210 may utilize at least any one carbon source selected from the group consisting of D-galactose, D-glucose, D-fructose, D-mannose, dulcitol, mannitol, sorbitol, N-acetyl-glucosamine, amygdalin, arbutin, esculin, salicin, cellobiose, maltose, lactose, trehalose, melezitose, gentiobiose, D-tagatose, gluconate, and L-fucose.

In another aspect, the present disclosure provides *Bifidobacterium longum* NK219 (Depository Institution: Korean Culture Center of Microorganisms, Deposit Date: September 15, 2021, Accession Number: KCCM13050P).

*Bifidobacterium longum* NK219 of the present disclosure is characterized as a novel lactic acid bacterium of *Bifidobacterium longum* isolated and identified from human feces.

The 16S rDNA sequence for identification and classification of *Bifidobacterium longum* NK219 of the present disclosure is the same as SEQ ID NO: 2 attached to the present specification. Therefore, *Bifidobacterium longum* NK219 of the present disclosure may comprise 16S rDNA of SEQ ID NO: 2.

Analysis of the 16S rDNA sequence of SEQ ID NO: 2 showed 99% homology with known *Bifidobacterium longum* strains, indicating the highest molecular phylogenetic relationship with *Bifidobacterium longum.* Therefore, the lactic acid bacterium was identified as *Bifidobacterium longum,* named *Bifidobacterium longum* NK219, and deposited at the Korean Culture Center of Microorganisms on September 15, 2021 (Accession Number: KCCM13050P).

*Bifidobacterium longum* NK219 of the present disclosure is a Gram-positive bacterium, and its cell morphology is bacillus. More specific physiological characteristics of *Bifidobacterium longum* NK219 may be analyzed according to conventional methods in the art, and the results are shown in Table 4 below. Specifically, *Bifidobacterium longum* NK219 may utilize at least any one carbon source selected from the group consisting of D-glucose, D-lactose, D-saccharose, D-maltose, salicin, D-xylose, L-arabinose, gelatin, D-mannose, D-raffinose, and D-trehalose.

In still another aspect, the present disclosure provides *Lactococcus lactis* NK209 (Depository Institution: Korean Culture Center of Microorganisms, Deposit Date: September 15, 2021, Accession Number: KCCM13048P).

*Lactococcus lactis* NK209 of the present disclosure is characterized as a novel lactic acid bacterium of *Lactococcus lactis* isolated and identified from human feces.

The 16S rDNA sequence for identification and classification of *Lactococcus lactis* NK209 of the present disclosure is the same as SEQ ID NO: 3 attached to the present specification. Therefore, *Lactococcus lactis* NK209 of the present disclosure may comprise 16S rDNA of SEQ ID NO: 3.

Analysis of the 16S rDNA sequence of SEQ ID NO: 3 showed 98% homology with known *Lactococcus lactis* strains, indicating the highest molecular phylogenetic relationship with *Lactococcus lactis.* Therefore, the lactic acid bacterium was identified as *Lactococcus lactis,* named *Lactococcus lactis* NK209, and deposited at the Korean Culture Center of Microorganisms on September 15, 2021 (Accession Number: KCCM13048P).

*Lactococcus lactis* NK209 of the present disclosure is a Gram-positive bacterium, and its cell morphology is bacillus. More specific physiological characteristics of *Lactococcus lactis* NK209 may be analyzed according to conventional methods in the art, and the results are shown in Table 5 below. Specifically, *Lactococcus lactis* NK209 may utilize at least any one carbon source selected from the group consisting of D-ribose, D-galactose, D-glucose, D-fructose, D-mannose, mannitol, esculin, salicin, cellobiose, maltose, melibiose, sucrose, trehalose, raffinose, starch, gentiobiose, N-acetyl-glucosamine, amygdalin and arbutin.

The present disclosure provides a composition comprising *Lactobacillus rhamnosus* NK210 KCCM13049P, *Bifidobacterium longum* NK219 KCCM13050P, *Lactococcus lactis* NK209 KCCM13048P, or any mixture thereof.

The present disclosure provides a composition for immunoregulation comprising *Lactobacillus rhamnosus* NK210 KCCM13049P, *Bifidobacterium longum* NK219 KCCM13050P, *Lactococcus lactis* NK209 KCCM13048P, or any mixture thereof.

The present disclosure provides a composition for enhancing immunity comprising *Lactobacillus rhamnosus* NK210 KCCM13049P, *Bifidobacterium longum* NK219 KCCM13050P, *Lactococcus lactis* NK209 KCCM13048P, or any mixture thereof.

The present disclosure provides a composition for improving immune function comprising *Lactobacillus rhamnosus* NK210 KCCM13049P, *Bifidobacterium longum* NK219 KCCM13050P, *Lactococcus lactis* NK209 KCCM13048P, or any mixture thereof.

In still another aspect, the present disclosure provides a pharmaceutical composition comprising *Lactobacillus rhamnosus* NK210 KCCM13049P, *Bifidobacterium longum* NK219 KCCM13050P, *Lactococcus lactis* NK209 KCCM13048P, or any mixture thereof.

In the present disclosure, the pharmaceutical composition may be a pharmaceutical composition comprising one, two, or three of the above-described probiotics.

In still another aspect, the present disclosure provides a pharmaceutical composition comprising *Lactobacillus rhamnosus* NK210 KCCM13049P, *Bifidobacterium longum* NK219 KCCM13050P, *Lactococcus lactis* NK209 KCCM13048P, or any mixture thereof, and a pharmaceutically acceptable carrier.

In still another aspect, the present disclosure provides a pharmaceutical composition for preventing or treating an inflammatory disease comprising *Lactobacillus rhamnosus* NK210 KCCM13049P, *Bifidobacterium longum* NK219 KCCM13050P, *Lactococcus lactis* NK209 KCCM13048P, or any mixture thereof.

In still another aspect, the present disclosure provides a pharmaceutical composition for preventing or treating cognitive dysfunction comprising *Lactobacillus rhamnosus* NK210 KCCM13049P, *Bifidobacterium longum* NK219 KCCM13050P, *Lactococcus lactis* NK209 KCCM13048P, or any mixture thereof.

The present disclosure provides a pharmaceutical composition for immunoregulation comprising *Lactobacillus rhamnosus* NK210 KCCM13049P, *Bifidobacterium longum* NK219 KCCM13050P, *Lactococcus lactis* NK209 KCCM13048P, or any mixture thereof.

The present disclosure provides a pharmaceutical composition for enhancing immunity comprising *Lactobacillus rhamnosus* NK210 KCCM13049P, *Bifidobacterium longum* NK219 KCCM13050P, *Lactococcus lactis* NK209 KCCM13048P, or any mixture thereof.

The present disclosure provides a pharmaceutical composition for improving immune function comprising *Lactobacillus rhamnosus* NK210 KCCM13049P, *Bifidobacterium longum* NK219 KCCM13050P, *Lactococcus lactis* NK209 KCCM13048P, or any mixture thereof.

Specifically, the compositions for enhancing immunity and/or improving immune function are intended to enhance human or animal health and may be administered to a healthy human or animal.

Further, the compositions for enhancing immunity and/or improving immune function are intended to enhance and/or improve the immunity of an immunocompromised human or animal, e.g., an immunocompromised human or animal due to radiation exposure, antibiotic or anticancer drug administration, and may be administered to any of the above-described immunocompromised humans or animals.

As used herein, an inflammatory disease is a collective term for a disease in which inflammation is the primary lesion and may be at least one selected from the group consisting of inflammatory bowel disease, arthritis, gout, hepatitis, asthma, obesity, keratitis, gastritis, nephritis, colitis, diabetes, tuberculosis, bronchitis, pleurisy, peritonitis, spondylitis, pancreatitis, urethritis, cystitis, vaginitis, arteriosclerosis, sepsis, burns, dermatitis, periodontitis, and gingivitis.

The term inflammatory bowel disease (IBD) refers to a class of inflammatory conditions of the colon and gastrointestinal tract. The main types of IBD are ulcerative colitis (UC) and Crohn's disease. The main difference between UC and Crohn's disease is the location and nature of the inflammatory changes. Crohn's disease may affect any part of the gastrointestinal tract, from the mouth to the anus, while UC is limited to the colon and rectum. Due to the nature of the presentation, a definitive diagnosis of Crohn's disease or UC cannot be made. In these cases, a diagnosis of indeterminate colitis may be made. Other forms of IBD include, but are not limited to, collagenous colitis, lymphocytic colitis, ischemic colitis, diversion colitis, Behcet's disease, indeterminate colitis, antibiotic-induced colitis, and anticancer drug-induced colitis.

In other words, the inflammatory bowel disease according to the present disclosure may be at least one selected from the group consisting of ulcerative colitis, Crohn's disease, collagenous colitis, lymphocytic colitis, ischemic colitis, diversion colitis, Behcet's disease, indeterminate colitis, antibiotic-induced colitis, and anticancer drug-induced colitis.

Antibiotic-induced colitis occurs after antibiotic administration and is caused by disruption of the normal bacterial flora present in the intestinal tract following exposure to antibiotics. Normal bacterial flora ferment unabsorbed carbohydrates in the intestinal tract to produce short chain fatty acids, but when antibiotics reduce the normal bacterial flora and disrupt the fermentation of carbohydrates, the osmotic pressure and acidity in the intestinal tract change, causing diarrhea and intestinal inflammation. Approximately 20% of antibiotic-induced colitis is caused by *C*. *difficile* overgrowth, and in a small number of cases, *C. perfringens, S. aureus, E. coli,* and *Candida albicans* may also be responsible. Examples of these antibiotics may include clindamycin, ampicillin, amoxicillin, cephalosporins, chloramphenicol, and erythromycin, etc.

Anticancer drug-induced colitis is an inflammatory condition that occurs as a side effect in the colon caused by the administration of anticancer drugs. For example, it may be caused by anticancer drugs such as 5-fluorouracil, cyclophosphamide, capecitabine, oxaliplatin, irinotecan, cetuximab, and bevacizumab.

As used herein, cognitive dysfunction refers to a disorder characterized by cognitive impairment and behavioral changes, resulting from a decrease in functions such as memory, spatial perception, judgment, executive function, and language. The cognitive dysfunction may be selected from, but is not limited to, the group consisting of, for example, anxiety, depression, migraine, stress, Alzheimer's disease, Huntington's disease, vascular dementia, Pick's disease, Parkinson's disease, Creutzfeldt-Jakob disease, dementia, and a combination thereof.

As used herein, "neuroinflammation" refers to inflammation in the brain, which is an important factor in the development of cognitive dysfunction-related diseases. It is known that over-activation of inflammatory cells in the brain leads to increased secretion of pro-inflammatory cytokines, and that cognitive dysfunction is caused by brain cell damage due to over-activation of this inflammatory response.

In the present disclosure, improving immune function means activating immunoregulation.

In this context, immunoregulation means resolving immune imbalances in the body and maintaining immune homeostasis. The maintenance of immune homeostasis refers to a state of balance between immune tolerance, which suppresses immunity, and immune response, which enhances immunity. Such immunoregulation may aim to improve immune function, preferably for the purpose of enhancing immunity. For example, it may be characterized as a supplement to the host's gut microflora, improving gut barrier function, and modulating the host's immune system.

More specifically, improving immune function as used herein may also refer to enhancing immunity, which may be interpreted to include immunoregulation effects in addition to its dictionary meaning. In other words, improving immune function and enhancing immunity are equally usable within the present disclosure.

Improving immune function and/or enhancing immunity may be to increase the activity of the immune system by increasing or regulating the activity of immune regulatory factors.

Specifically, it may be effective in preventing and improving immunodeficiency and preventing, improving, and treating immune-related dysfunction or related diseases by inhibiting neovascularization, increasing growth inhibition against pathogens, and the like.

Specifically, it is to enhance biological defense ability against an antigen, and may be effective in preventing and improving immunodeficiency and preventing, improving, and treating immune-related dysfunction or related diseases by increasing cellular and humoral immunity to antigens.

Mechanisms of improving immune function and/or enhancing immunity are not limited, but may include, for example, promoting the activity of antigen-presenting cells such as macrophages, promoting specific activity against lymphocytes, eliminating killer cells, or modulating the secretion of immune mediators.

The enhancement of immunity according to the present disclosure is not limited to, but may be for the enhancement of immunity in a subject with normal immunity, for example, to promote health.

Further, it may be intended to boost immunity that has been compromised by radiation therapy or exposure, antibiotic treatment, or anticancer drug treatment.

Specifically, it may be aimed at improving and/or recovering the occurrence of immunodeficiency, immunosuppression, or immune system damage due to administrations of antibiotics or anticancer drugs.

Anticancer drugs may be drugs such as compounds or proteins that promote cancer regression or further prevent tumor growth, and may also include other anticancer therapy, radiation therapy, and other anticancer therapies other than drug therapy. The other therapeutic agent may be, but is not limited to, an immune checkpoint inhibitor, a chemical anticancer agent, or a targeted anticancer agent.

Antibiotics include any antibiotic substances, including, but not limited to, clindamycin, ampicillin, amoxicillin, cephalosporin, chloramphenicol, and erythromycin.

In an embodiment of the present disclosure, *Lactobacillus rhamnosus* NK210 KCCM13049P, *Bifidobacterium longum* NK219 KCCM13050P, *Lactococcus lactis* NK209 KCCM13048P, or any mixture thereof, is effective in inhibiting inflammatory diseases and cognitive dysfunction, and improving immune function by showing excellent antioxidant activity.

In an embodiment of the present disclosure, *Lactobacillus rhamnosus* NK210 KCCM13049P, *Bifidobacterium longum* NK219 KCCM13050P, *Lactococcus lactis* NK209 KCCM13048P, or any mixture thereof, is effective in improving inflammation by regulating the expression of cytokines, such as TNF-α and IL-10.

In an embodiment of the present disclosure, *Lactobacillus rhamnosus* NK210 KCCM13049P, *Bifidobacterium longum* NK219 KCCM13050P, *Lactococcus lactis* NK209 KCCM13048P, or any mixture thereof, is effective in improving inflammation by regulating the expression of cytokines, such as by regulating the expression of pro-inflammatory cytokines, e.g., TNF-a, and increasing the expression of anti-inflammatory cytokines, e.g., IL-10.

In an embodiment of the present disclosure, *Lactobacillus rhamnosus* NK210 KCCM13049P, *Bifidobacterium longum* NK219 KCCM13050P, *Lactococcus lactis* NK209 KCCM13048P, or any mixture thereof, is effective in preventing and treating cognitive dysfunction by increasing the expression of brain-derived neurotrophic factor (BDNF) and increasing the expression of neuropeptide Y, which is highly associated with stress, anxiety, depression, and cognitive impairment.

In an embodiment of the present disclosure, *Lactobacillus rhamnosus* NK210 KCCM13049P, *Bifidobacterium longum* NK219 KCCM13050P, *Lactococcus lactis* NK209 KCCM13048P, or any mixture thereof, is effective in inhibiting inflammatory diseases, regulating immune function, and modulating nervous system and cognitive function through regulation of gut microbiota. Specifically, the lactic acid bacteria of the present disclosure regulate gut microbiota, including inhibiting *Clostridium difficile,* which is highly associated with enteritis, and reduce LPS levels in the gut. In addition, the lactic acid bacteria of the present disclosure improve intestinal health by promoting beneficial bacteria in the intestines and reducing harmful bacteria, and thus have an effect on improving nerves, especially cognitive function, through the Gut-Brain Axis.

In an embodiment of the present disclosure, administration of strains according to the present disclosure to an animal with LPS-induced cognitive impairment improved gut function, systemic inflammatory responses, and cognitive impairment, and reduced levels of inflammation in the nerves. Specifically, against inflammatory bowel diseases induced by *Escherichia coli-*derived LPS, the lactic acid bacteria of the present disclosure restored the decrease in intestinal length, reduced the activity of MPO, decreased the level of inflammatory cytokines, and increased the level of anti-inflammatory cytokines. In addition, in the systemic inflammatory response confirmed through the spleen, the phagocytic ability of macrophages and the killing ability of NK cells were controlled to reduce the level of inflammatory cytokines and increase the level of anti-inflammatory cytokines. It also exhibits behavioral properties for improving memory in the nervous system, especially for cognitive dysfunction, and also has excellent improvement effects on neuroinflammation.

According to an embodiment of the present disclosure, administration of strains according to the present disclosure to an animal model with anticancer drug-induced immune damage (inhibition) improved gut function, systemic inflammatory responses, immune function, and cognitive impairment, and reduced levels of inflammation in the nerves. Specifically, the animal model with impaired immune function caused by administration of anticancer drug restored the decrease in intestinal length, reduced the activity of MPO, and normalized the expression ratio of inflammatory cytokines and anti-inflammatory cytokines. In addition, in the systemic inflammatory response confirmed through the spleen, the phagocytic ability of macrophages and the killing ability of NK cells increased, and the level of anti-inflammatory cytokines increased compared to the level of inflammatory cytokines. It also exhibits behavioral properties for improving memory in the nervous system, especially for cognitive dysfunction, and also has excellent improvement effects on neuroinflammation.

According to an embodiment of the present disclosure, administration of strains according to the present disclosure to an animal model with antibiotic-induced gut microbiota imbalance improved gut function, systemic inflammatory responses, immune function, and cognitive impairment, and reduced levels of inflammation in the nerves. Specifically, the animal model with gut microbiota imbalance caused by administration of antibiotics such as ampicillin restored the decrease in intestinal length, reduced the activity of MPO, and normalized the expression ratio of inflammatory cytokines and anti-inflammatory cytokines. In addition, in the systemic inflammatory response confirmed through the spleen, the phagocytic ability of macrophages and the killing ability of NK cells were controlled to reduce the level of inflammatory cytokines and increase the level of anti-inflammatory cytokines. It also exhibits behavioral properties for improving memory in the nervous system, especially for cognitive dysfunction, and also has excellent improvement effects on neuroinflammation.

According to an embodiment of the present disclosure, administration of strains according to the present disclosure to a healthy animal model has been shown to have preventive or improvement effects including improvement of gut function, systemic inflammatory responses, immune function, and cognitive impairment.

According to an embodiment of the present disclosure, administration of strains according to the present disclosure to an animal model with TNBS-induced intestinal disease and cognitive dysfunction improved gut function, systemic inflammatory responses, immune function, and cognitive impairment, and reduced levels of inflammation in the nerves. Specifically, the animal model with TNBS-induced intestinal disease and cognitive dysfunction restored the decrease in intestinal length, reduced the activity of MPO, and normalized the expression ratio of inflammatory cytokines and anti-inflammatory cytokines. It also exhibits behavioral properties for improving memory in the nervous system, especially for cognitive dysfunction, and also has excellent improvement effects on neuroinflammation.

Specifically, the strains contained in the pharmaceutical composition of the present disclosure may be used in various forms such as live bacteria, dead bacteria, cultures, lysates, or extracts thereof.

Live bacteria refer to bacteria that are alive as they are.

Dead bacteria are a form in which live bacteria cultured under certain conditions are collected, and further growth of the bacteria is prevented by methods such as heat drying, pressurization, and drug treatment.

A culture refers to an object obtained by culturing lactic acid bacteria in a known liquid medium or solid medium, and includes strains according to the present disclosure. The product may contain lactic acid bacteria. The medium may be selected from known liquid media or solid media, and may be, for example, but not limited to, MRS liquid medium, GAM liquid medium, MRS agar medium, GAM agar medium, BL agar medium.

As used herein, the lysate means that live bacteria, dead bacteria, or cultures thereof are isolated and processed through mechanical or chemical methods to have a lysate form. For example, the lysate form may be produced through bead mills, presses, sonicators or microfluidizers, enzyme treatment, etc.

As used herein, the extract refers to the "water (liquid)" form obtained by extracting live and dead bacteria once more according to a commonly known extraction method. For example, the extract means a product obtained by extracting live bacteria, dead bacteria and/or lysates using a commonly known extraction method (e.g., obtained by extraction with a known extraction solvent such as water, C1 to C4 alcohol (methanol, ethanol, etc.)).

According to the present disclosure, the above-described strains may be used alone.

In addition, a combination of strains may achieve a synergistic effect, and the form of a combination of one or more types of strains may be employed.

These combination forms may include, for example,
*Lactobacillus rhamnosus* NK210 KCCM13049P and *Bifidobacterium longum* NK219 KCCM13050P;
*Lactobacillus rhamnosus* NK210 KCCM13049P and *Lactococcus lactis* NK209 KCCM13048P;
*Bifidobacterium longum* NK219 KCCM13050P and *Lactococcus lactis* NK209 KCCM13048P; or
*Lactobacillus rhamnosus* NK210 KCCM13049P, *Bifidobacterium longum* NK219 KCCM13050P, and *Lactococcus lactis* NK209 KCCM13048P.

Preferably, the combination form may be *Lactobacillus rhamnosus* NK210 KCCM13049P and *Bifidobacterium longum* NK219 KCCM13050P, or *Bifidobacterium longum* NK219 KCCM13050P and *Lactococcus lactis* NK209 KCCM13048P, or *Lactobacillus rhamnosus* NK210 KCCM13049P *Bifidobacterium longum* NK219 KCCM13050P, and *Lactococcus lactis* NK209 KCCM13048P.

As an example, the combination of *Lactobacillus rhamnosus* NK210 KCCM13049P and *Bifidobacterium longum* NK219 KCCM13050P strains may be, for example, 10:1, 9:1, 8:1, 7:1, 6 :1, 5:1, 4:1, 3:1, 2:1, 1:1, 1:2, 1:3, 1:4, 1:5, 1:6, 1:7, 1:8, 1:9 or 1:10, based on the number of bacteria (CFU: colony forming unit). Preferably, the strains may be combined at 9:1 to 1:1, more preferably at 4:1 to 1:1, and more specifically at 4:1.

As an example, the combination of *Lactococcus lactis* NK209 KCCM13048P and *Bifidobacterium longum* NK219 KCCM13050P strains may be, for example, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9 or 1:10, based on the number of bacteria (CFU: colony forming unit). Preferably, the strains may be combined at 9:1 to 1:1, more preferably at 4:1 to 1:1, and more specifically at 4:1.

As an example, the combination of *Lactococcus lactis* NK209 KCCM13048P and *Lactobacillus rhamnosus* NK210 KCCM13049P strains may be, for example, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9 or 1:10, based on the number of bacteria (CFU: colony forming unit). Preferably, the strains may be combined at 9:1 to 1:1, more preferably at 4:1 to 1:1.

As an example, in the case of three types of combination, the three types of strains may be combined at, for example, 4-8: 4-8:1-2 (NK209:NK210:NK219) based on the number of bacteria (CFU), and preferably, 2:2:1.

The pharmaceutical compositions according to the present disclosure may be prepared into pharmaceutical formulations using methods well known in the art to provide rapid, sustained or delayed release of active ingredients after being administered to a mammal. In the preparation of the formulation, the pharmaceutical composition according to the present disclosure may further comprise a pharmaceutically acceptable carrier within the range that does not inhibit the activity of the novel lactic acid bacteria.

Examples of the pharmaceutically acceptable carrier may include, but not limited to, those commonly used, such as lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. In addition, the pharmaceutical composition of the present disclosure may contain diluents or excipients such as fillers, extenders, binders, wetting agents, disintegrants, surfactants, and other pharmaceutically acceptable additives.

The dosage of the pharmaceutical composition according to the present disclosure must be a pharmaceutically effective amount. The term "pharmaceutically effective amount" means an amount sufficient to prevent or treat the above-described disease or condition at a reasonable benefit/risk ratio applicable to medical treatment. The effective dose level may be varied by those skilled in the art depending on factors such as method of formulation, patient condition and weight, patient gender, age, severity of disease, drug form, route and duration of administration, rate of excretion, response sensitivity, etc. The effective amount may vary depending on the route of processing, the use of excipients and the possibility of use with other agents, as recognized by those skilled in the art. However, for desirable effects, in the case of oral administration, the composition of the present disclosure may be administered to an adult at an amount of 0.0001 to 100 mg/kg of body weight per day, preferably 0.001 to 100 mg/kg per day. The dose may be administered once daily or in several divided doses. The above dosages are not intended to limit the scope of the present disclosure in any way.

The pharmaceutical composition of the present disclosure may be administered to mammals, such as mice, livestock, and humans, by a variety of routes. Specifically, the pharmaceutical compositions of the present disclosure may be administered orally or parenterally (e.g., by application or intravenous, subcutaneous, or intraperitoneal injection), but oral administration is preferred. Solid preparations for oral administration may include powders, granules, tablets, capsules, soft capsules, pills, etc. Liquid preparations for oral administration may include suspensions, solutions for internal use, emulsions, syrups, and aerosols, and may include various excipients such as wetting agents, sweeteners, flavors, preservatives, and the like, in addition to water and liquid paraffin that are commonly used simple diluents. Preparations for parenteral administration may be used by formulation in the form of aqueous solutions, liquids, non-aqueous solutions, suspensions, emulsions, eye drops, eye ointments, syrups, suppositories, aerosols, and the like, for external use, each sterilized according to conventional methods, or sterile injectable preparations, and preferably, but not limited to, may be used by preparing pharmaceutical compositions of creams, gels, poultices, sprays, ointments, plasters, lotions, liniments, ointments, eye drops, pastes or cataplasms. Formulations for topical administration may be anhydrous or aqueous, depending on clinical prescription. As the non-aqueous solvents and the suspensions, propylene glycol, polyethylene glycol, vegetable oils such as olive oil, and injectable esters such as ethyl oleate, and the like, may be used. As the base of the suppository, witepsol, macrogol, Tween 61, cacao butter, laurin butter, glycerogelatin and the like, may be used.

In still another aspect, the present disclosure provides a method for preventing or treating cognitive dysfunction, comprising administering to a subject *Lactobacillus rhamnosus* NK210 KCCM13049P, *Bifidobacterium longum* NK219 KCCM13050P, *Lactococcus lactis* NK209 KCCM13048P, or any mixture thereof.

In still another aspect, the present disclosure provides a method for preventing or treating an inflammatory disease, comprising administering to a subject *Lactobacillus rhamnosus* NK210 KCCM13049P, *Bifidobacterium longum* NK219 KCCM13050P, *Lactococcus lactis* NK209 KCCM13048P, or any mixture thereof.

In still another aspect, the present disclosure provides a method for immunoregulation, comprising administering to a subject *Lactobacillus rhamnosus* NK210 KCCM13049P, *Bifidobacterium longum* NK219 KCCM13050P, *Lactococcus lactis* NK209 KCCM13048P, or any mixture thereof.

In still another aspect, the present disclosure provides a method for improving immune function, comprising administering to a subject *Lactobacillus rhamnosus* NK210 KCCM13049P, *Bifidobacterium longum* NK219 KCCM13050P, *Lactococcus lactis* NK209 KCCM13048P, or any mixture thereof.

In still another aspect, the present disclosure provides a method for enhancing immune, comprising administering to a subject *Lactobacillus rhamnosus* NK210 KCCM13049P, *Bifidobacterium longum* NK219 KCCM13050P, *Lactococcus lactis* NK209 KCCM13048P, or any mixture thereof.

In the present disclosure, descriptions of the above-described strains, diseases, administration, etc., may appropriately reflect the above-described contents.

The subject refers to an animal, typically a mammal capable of showing beneficial effects from treatment with the novel probiotics of the present disclosure. Preferred examples of such subjects may include primates such as humans.

The present disclosure provides *Lactobacillus rhamnosus* NK210 KCCM13049P, *Bifidobacterium longum* NK219 KCCM13050P, *Lactococcus lactis* NK209 KCCM13048P, or any mixture thereof, for use in the prevention or treatment of an inflammatory disease.

The present disclosure provides *Lactobacillus rhamnosus* NK210 KCCM13049P, *Bifidobacterium longum* NK219 KCCM13050P, *Lactococcus lactis* NK209 KCCM13048P, or any mixture thereof, for use in the prevention or treatment of cognitive dysfunction.

The present disclosure provides *Lactobacillus rhamnosus* NK210 KCCM13049P, *Bifidobacterium longum* NK219 KCCM13050P, *Lactococcus lactis* NK209 KCCM13048P, or any mixture thereof, for use in immunoregulation.

The present disclosure provides *Lactobacillus rhamnosus* NK210 KCCM13049P, *Bifidobacterium longum* NK219 KCCM13050P, *Lactococcus lactis* NK209 KCCM13048P, or any mixture thereof, for use in improving immune function.

The present disclosure provides *Lactobacillus rhamnosus* NK210 KCCM13049P, *Bifidobacterium longum* NK219 KCCM13050P, *Lactococcus lactis* NK209 KCCM13048P, or any mixture thereof, for use in enhancing immunity.

The present disclosure provides use of *Lactobacillus rhamnosus* NK210 KCCM13049P, *Bifidobacterium longum* NK219 KCCM13050P, *Lactococcus lactis* NK209 KCCM13048P, or any mixture thereof, in the manufacture of a medicament for the treatment of an inflammatory disease.

The present disclosure provides use of *Lactobacillus rhamnosus* NK210 KCCM13049P, *Bifidobacterium longum* NK219 KCCM13050P, *Lactococcus lactis* NK209 KCCM13048P, or any mixture thereof, in the manufacture of a medicament for the treatment of cognitive dysfunction.

The present disclosure provides use of *Lactobacillus rhamnosus* NK210 KCCM13049P, *Bifidobacterium longum* NK219 KCCM13050P, *Lactococcus lactis* NK209 KCCM13048P, or any mixture thereof, in the manufacture of a medicament for use in immunoregulation.

The present disclosure provides use of *Lactobacillus rhamnosus* NK210 KCCM13049P, *Bifidobacterium longum* NK219 KCCM13050P, *Lactococcus lactis* NK209 KCCM13048P, or any mixture thereof, in the manufacture of a medicament for use in improving immune function.

The present disclosure provides use of *Lactobacillus rhamnosus* NK210 KCCM13049P, *Bifidobacterium longum* NK219 KCCM13050P, *Lactococcus lactis* NK209 KCCM13048P, or any mixture thereof, in the manufacture of a medicament for use in enhancing immunity.

In another aspect, the present disclosure provides a food composition for preventing or improving an inflammatory disease, comprising *Lactobacillus rhamnosus* NK210 KCCM13049P, *Bifidobacterium longum* NK219 KCCM13050P, *Lactococcus lactis* NK209 KCCM13048P, or any mixture thereof.

In another aspect, the present disclosure provides a food composition for preventing or improving cognitive dysfunction, comprising *Lactobacillus rhamnosus* NK210 KCCM13049P, *Bifidobacterium longum* NK219 KCCM13050P, *Lactococcus lactis* NK209 KCCM13048P, or any mixture thereof.

In another aspect, the present disclosure provides a food composition for immunoregulation, comprising *Lactobacillus rhamnosus* NK210 KCCM13049P, *Bifidobacterium longum* NK219 KCCM13050P, *Lactococcus lactis* NK209 KCCM13048P, or any mixture thereof.

In another aspect, the present disclosure provides a food composition for improving immune function, comprising *Lactobacillus rhamnosus* NK210 KCCM13049P, *Bifidobacterium longum* NK219 KCCM13050P, *Lactococcus lactis* NK209 KCCM13048P, or any mixture thereof.

The present disclosure provides a food composition for enhancing immunity, comprising *Lactobacillus rhamnosus* NK210 KCCM13049P, *Bifidobacterium longum* NK219 KCCM13050P, *Lactococcus lactis* NK209 KCCM13048P, or any mixture thereof.

Health functional food is defined as a category of food that emphasizes the bioregulatory function of food, incorporating added value through the utilization of physical, biochemical, and biotechnological methods to act and express specific purposes. The food compositions of the present disclosure may be utilized as health functional food. The ingredients of such health functional food are designed and processed to exert in vivo regulatory functions on the body that are related to biological defense, regulation of body rhythms, and prevention and recovery from disease, and may encompass dietary supplements, sweeteners, or functional ingredients that are acceptable as food.

When the strains of the present disclosure are to be used as health functional food (or health functional beverage additives), the novel strains may be added as they are or in combination with other foods or food ingredients and used as appropriate according to conventional methods. The mixing amount of the strains may be suitably determined depending on the purpose of its use (prevention, health or improvement, therapeutic treatment).

Preferably, the strains according to the present disclosure may be in the form of a single, two- or three-typed mixture. Within the above-described scope, the utilization of mixed strains may be contemplated, considering potential synergistic effects.

The food may contain various nutrients, vitamins, minerals (electrolytes), synthetic and natural flavoring agents, colorants, and enhancers (cheese, chocolate, etc.), pectic acid and salts thereof, organic acids, protective colloidal thickeners, pH adjusters, stabilizers, preservatives, glycerin, alcohol, carbonation agents used in carbonated beverages, and the like. In addition, the health functional food of the present disclosure may contain pulp for the manufacture of fruit and vegetable drinks. These ingredients may be used alone or in combination, and the proportions of these additives are typically selected in the range of 0.001 to 50 parts by weight of the total weight of the composition.

There are no specific restrictions on the types of foods. Foods to which the strains may be added include sausages, meats, breads, chocolates, snacks, candies, confections, ramen, pizza, other noodles, chewing gum, dairy products including ice cream, various soups, beverages, teas, drinks, alcoholic beverages, and vitamin complexes. When formulated into a beverage, the liquid ingredients added in addition to the novel lactic acid bacteria may include, but are not limited to, various flavoring agents or natural carbohydrates as additional ingredients, as in conventional beverages. The above-described natural carbohydrates include monosaccharides (e.g. glucose, fructose, etc.), disaccharides (e.g. maltose, sucrose, etc.), polysaccharides (e.g. conventional sugars such as dextrins, cyclodextrins, etc.), and sugar alcohol such as xylitol, sorbitol, erythritol, etc.

The numerical values provided in this specification should be construed to include the equivalent range unless explicitly stated otherwise.

The following Examples are presented to enhance the understanding of the present disclosure. These Examples are only provided to more easily understand the present disclosure, and do not impose limitations on the content of the present disclosure.

### [Advantageous Effects]

The novel probiotics according to the present disclosure have high utility as a medicine or food and, in particular, can be used for preventing, treating, or relieving inflammatory diseases and cognitive dysfunction and for improving immune function, due to having an excellent antioxidant effect, inflammation-relieving effect, nerve-improving effect, cognition-improving effect, gut microbiota imbalance-improving effect, immune-regulating effect, etc.

### [Description of Drawings]

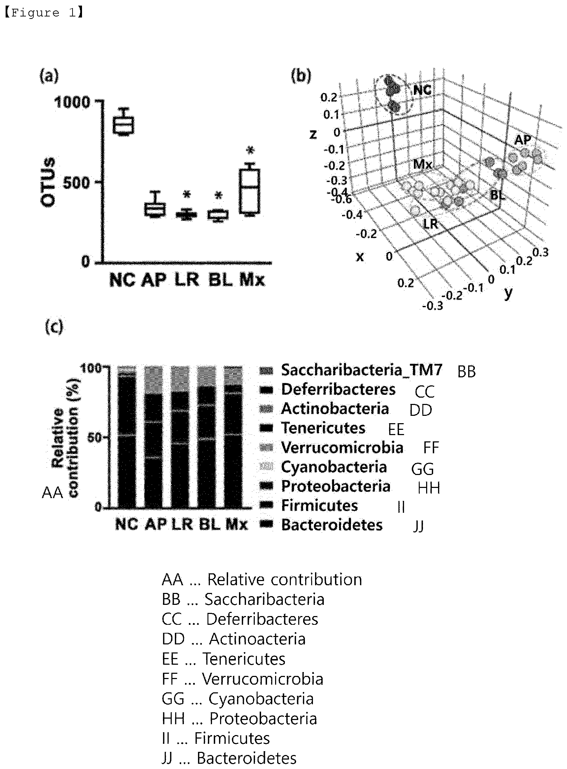

FIG. 1 shows results confirming the improvement in ampicillin-induced gut microbiota imbalance, through strain treatments according to the present disclosure (AP: Ampicillin, LR: *Lactobacillus rhamnosus* NK210, BL: *Bifidobacterium longum* NK219, and Mx: NK210+NK219).

### [Best Mode]

The following Examples are presented to enhance comprehension of the present disclosure. These Examples are only provided to more easily understand the present disclosure, and do not impose limitations on the content of the present disclosure.

### Example 1: Isolation and identification of lactic acid bacteria

### (1) Isolation of lactic acid bacteria from human feces

Human feces were suspended in GAM liquid medium (GAM broth; Nissui Pharmaceutical, Japan) and allowed to stand at 4°C for 10 minutes, and the supernatant was extracted and transferred to MRS agar medium (BD, USA), incubated anaerobically at 37°C for about 48 hours, and subsequently, the grown colonies were isolated.

### (2) Identification of selected lactic acid bacteria

The strains isolated were subjected to analysis, encompassing both physiological characteristics and 16S rDNA sequences thereof. Moreover, the carbon source availability among the physiological characteristics was analyzed with the API Kit (model name: API 50 CHL; manufacturer: BioMerieuxs, USA) to determine the species of the strains and assign strain names.

The strains identified through the above procedures are listed in Table 1 below.

**[Table 1]**

| No. | Scientific name | No. | Scientific name |
|---|---|---|---|
| 1 | *Lactobacillus plantarum* NK181 | 26 | *Lactococcus graminis* NK206 |
| 2 | *Lactobacillus plantarum* NK182 | 27 | *Lactococcus graminis* NK207 |
| 3 | *Lactobacillus plantarum* NK183 | 28 | *Lactococcus lactis* NK208 |
| 4 | *Lactobacillus plantarum* NK184 | 29 | *Lactococcus lactis* NK209 |
| 5 | *Lactobacillus gasseri* NK185 | 30 | *Lactobacillus rhamnosus* NK210 |
| 6 | *Lactobacillus gasseri* NK186 | 31 | *Lactobacillus rhamnosus* NK211 |
| 7 | *Lactobacillus gasseri* NK187 | 32 | *Lactobacillus rhamnosus* NK212 |
| 8 | *Lactobacillus gasseri* NK188 | 33 | *Bifidobacterium infantis* NK213 |
| 9 | *Lactobacillus paracasei* NK189 | 34 | *Bifidobacterium infantis* NK214 |
| 10 | *Lactobacillus paracasei* NK190 | 35 | *Bifidobacterium infantis* NK215 |
| 11 | *Lactobacillus paracasei* NK191 | 36 | *Bifidobacterium longum* NK216 |
| 12 | *Lactobacillus paracasei* NK192 | 37 | *Bifidobacterium longum* NK217 |
| 13 | *Lactobacillus casei* NK193 | 38 | *Bifidobacterium longum* NK218 |
| 14 | *Lactobacillus casei* NK194 | 39 | *Bifidobacterium longum* NK219 |
| 15 | *Lactobacillus casei* NK195 | 40 | *Bifidobacterium adolescentis* NK220 |
| 16 | *Lactobacillus casei* NK196 | 41 | *Bifidobacterium adolescentis* NK221 |
| 17 | Lactobacillus sakei NK197 | 42 | *Bifidobacterium adolescentis* NK222 |
| 18 | *Lactobacillus sakei* NK198 | 43 | *Bifidobacterium bifidum* NK223 |
| 19 | *Lactobacillus sakei* NK199 | 44 | *Bifidobacterium bifidum* NK224 |
| 20 | *Lactobacillus reuteri* NK200 | 45 | *Bifidobacterium catenulatum* NK225 |
| 21 | *Lactobacillus reuteri* NK201 | 46 | *Bifidobacterium catenulatum* NK226 |
| 22 | *Lactobacillus johnsonii* NK202 | 47 | Bifidobacterium catenulatum NK227 |
| 23 | *Lactobacillus johnsonii* NK203 | 48 | *Bifidobacterium pseudocatenulatum* NK228 |
| 24 | *Lactobacillus mucosae* NK204 | 49 | *Bifidobacterium pseudocatenulatum* NK229 |
| 25 | *Lactobacillus mucosae* NK205 | 50 | *Bifidobacterium pseudocatenulatum* NK230 |

### Example 2: Analysis on characterization of strains

A total of 50 strains isolated in Example 1 above were confirmed to have their antioxidant activity, changes in cytokine expression in macrophages, changes in BDNF expression, changes in NPY expression, and effects on *Clostridium difficile* proliferation and LPS production in the gut microbiota, thereby selecting strains with excellent characteristics. The specific experimental methods and measurement results are as follows.

### (1) Effects on antioxidant activity (in vitro)

DPPH (2,2-Diphenyl-1-picrylhydrazyl) was dissolved in ethanol to a concentration of 0.2 mM to prepare DPPH solution. Lactic acid bacteria suspension (1×10⁸ CFU/ml) or vitamin C solution (1 g/ml) was added to 0.1 ml of the above DPPH solution and incubated at 37°C for 20 min. The culture was centrifuged at 3000 rpm for 5 min to obtain a supernatant. The absorbance of the supernatant was then measured at 517 nm and the antioxidant activity of the lactic acid bacteria was calculated.

### (2) Macrophage isolation and effect on expression amount of inflammatory indicators TNF-α and IL-10

C57BL/6 mice (male, 6 weeks old, 20-23 g) were subjected to an intraperitoneal injection of 2 ml of sterile 4% thioglycolate solution, and after 96 hours, the mice were anesthetized and injected with 8 ml of RPMI 1640 medium through the peritoneal cavity. Then, after 5-10 minutes, the RPMI medium (macrophages) in the mouse peritoneal cavity was extracted, centrifuged at 1000 g for 10 minutes, and washed again with RPMI 1640 medium twice. Macrophages were seeded in 24-well plates at a count of 0.5×10⁶ in each well, and treated with lactic acid bacteria and LPS, an inflammatory response-inducing substance, for 24 hours. TNF-α and IL-10 cytokine expression amount in the culture supernatant was then measured by ELISA Kit.

### (3) Effect on BDNF expression in SH-SY5Y cells

SH-SY5Y cells obtained from the Korean Cell Line Bank were cultured in DMEM medium supplemented with 10% FBS and 1% antibiotics, and dispensed at 2 × 10⁶ cells per well in a 12-well plate. Then, LPS (200 ng/ml) was added to each well along with lactic acid bacteria (1×10⁴ CFU/ml) and incubated for 24 h. The cells and supernatants were collected, sonicated and centrifuged, and the amount of BDNF in the supernatant was measured by ELISA kit.

### (4) Effect on neuropeptide Y (NPY) expression in PC12 phaeochromocytoma cells

PC12 cells obtained from the Korean Cell Line Bank were cultured in DMEM medium supplemented with 10% FBS and 1% antibiotics, and dispensed at 1 × 10⁶ cells per well in a 12-well plate. Then, LPS (200 ng/ml) was added to each well along with lactic acid bacteria (1×10⁴ CFU/ml) and incubated for 24 hr. The cells and supernatants were collected, sonicated and centrifuged, and the amount of NPY in the supernatant was measured by ELISA kit.

### (5) Effect on Clostridium difficile proliferation and LPS production in gut microbiota

Fresh human feces were suspended in anaerobic medium (GAM, Nissui Pharmaceutical Co., Ltd., Japan) and allowed to stand for 10 min. The supernatant was diluted with 10,000-fold anaerobic medium, 0.05 mL of the dilution was transplanted into 5 mL of freshly made anaerobic medium, and lactic acid bacteria (1×10⁶ CFU/mL) were implanted and incubated anaerobically for 24 hr. This culture medium was divided into two, one was centrifuged (5000 rpm, 20 minutes), the precipitated DNA was isolated using the QIAamp DNA stool mini kit (Qiagen, Germany), and *Clostridium difficile* (primer: forward, 5'- GGG AGC TTC CCA TAC GGG TTG-3' (SEQ ID NO: 4); reverse, 5'-TTG ACT GCC TCA ATG CTT GGG C-3' (SEQ ID NO: 5) was quantified by qPCR. The reaction product was first treated at 95°C for 30 seconds, followed by 42 cycles of 5 seconds at 95°C and 30 seconds at 72°C for analysis. In addition, one culture medium was sonicated, centrifuged, and filter-sterilized, and the amount of LPS in the supernatant was measured using an ELISA kit.

Results thereof are shown in Table 2.

**[Table 2]**

| | Antioxid ant activity | Macrophage | | SHSY5Y cell | PC12 cell | Gut microbiota | |
|---|---|---|---|---|---|---|---|
| | | Inhibi tion of TNF-α expres sion | Induct ion of IL-10 expres sion | Induction of BDNF expressio n | Inducti on of NPY express ion | Inhibit ion of diffici le bacteri a prolife ration | Inhibit ion of LPS product ion |
| NK181 | + | + | - | - | + | + | - |
| NK182 | ++ | - | - | - | - | ++ | - |
| NK183 | + | + | - | - | - | + | + |
| NK184 | ++ | + | + | - | - | + | + |
| NK185 | ++ | + | + | - | + | + | - |
| NK186 | + | + | + | - | - | + | + |
| NK187 | + | + | - | + | - | + | + |
| NK188 | + | + | + | - | - | + | - |
| NK189 | + | + | - | - | - | + | + |
| NK190 | + | - | - | - | + | + | - |
| NK191 | + | - | + | + | + | - | - |
| NK192 | ++ | - | - | - | - | - | - |
| NK193 | + | + | + | + | - | - | + |
| NK194 | ++ | - | + | + | + | + | + |
| NK195 | + | + | + | - | - | + | - |
| NK196 | ++ | - | - | - | + | + | + |
| NK197 | ++ | + | + | - | + | + | + |
| NK198 | ++ | - | - | - | + | - | ++ |
| NK199 | + | + | | - | - | - | - |
| NK200 | + | - | - | + | + | + | + |
| NK201 | + | + | + | + | + | + | + |
| NK202 | + | + | + | + | + | + | + |
| NK203 | + | + | + | - | - | - | - |
| NK204 | + | - | - | - | - | + | - |
| NK205 | + | - | - | - | + | - | - |
| NK206 | + | - | - | | + | | |
| NK207 | - | | | - | - | + | + |
| NK208 | - | - | - | + | + | + | + |
| NK209 | + | + | + | + | + | +++ | + |
| NK210 | +++ | ++ | +++ | ++ | +++ | +++ | +++ |
| NK211 | + | + | + | - | - | + | - |
| NK212 | ++ | - | - | - | - | - | + |
| NK213 | + | + | + | - | - | + | - |
| NK214 | ++ | - | - | - | + | + | + |
| NK215 | ++ | + | + | - | + | + | + |
| NK216 | ++ | - | - | - | + | - | ++ |
| NK217 | + | + | | - | - | - | - |
| NK218 | + | - | - | + | + | + | + |
| NK219 | +++ | +++ | +++ | +++ | ++ | +++ | +++ |
| NK220 | + | + | + | + | - | + | - |
| NK221 | + | - | - | - | + | + | + |
| NK222 | + | + | + | - | + | + | + |
| NK223 | + | + | + | + | - | - | - |
| NK224 | + | - | - | - | + | + | + |
| NK225 | + | + | + | - | - | + | - |
| NK226 | + | - | - | + | - | + | - |
| NK227 | + | + | + | - | + | + | + |
| NK228 | + | - | - | + | + | - | ++ |
| NK229 | + | + | | + | - | - | - |
| NK230 | + | - | - | + | + | + | + |

| | | | | | | | |
|---|---|---|---|---|---|---|---|
| * very strongly (+++; >90%); strongly (++; >60-90%); weakly (+; >20-60%); not or less than 20%(-; <20%) | | | | | | | |

As shown in Table 2, both *Lactobacillus rhamnosus* NK210 and *Bifidobacterium longum* NK219 were found to exhibit all of excellent antioxidant activity, anti-inflammatory efficacy, neurological enhancement, and inhibition in proliferation of intestinal *Clostridium difficile* and LPS production, and the like.

Further, in addition to the strains, it was confirmed that the *Lactococcus lactis* NK209 strain exhibited activity above a certain level and, in particular, exhibited a significant effect in inhibiting the proliferation of intestinal *Clostridium difficile.*

Accordingly, based on the above results, additional experiments were conducted focusing on *Lactobacillus rhamnosus* NK210, *Bifidobacterium longum* NK219, and *Lactococcus lactis* NK209 strains.

The 16S rDNA sequence of *Lactobacillus rhamnosus* NK210 is specifically described in SEQ ID NO: 1, the 16s rDNA sequence of *Bifidobacterium longum* NK219 is specifically described in SEQ ID NO: 2, and the 16s rDNA sequence of *Lactococcus lactis* NK209 is specifically described in SEQ ID NO: 3. In addition, it was confirmed through the phylogenetic tree that these strains were all novel strains.

The present inventors have deposited a patent of *Lactobacillus rhamnosus* NK210, *Bifidobacterium longum* NK219, and *Lactococcus lactis* NK209 into the Korean Culture Center of Microorganisms, accredited depository institution (Address: Yulim Building, 45, 2ga-gil, Hongjenae, Seodaemungu, Seoul, Korea), and obtained accession numbers of KCCM13049P, KCCM13050P, and KCCM13048P, respectively, on September 15, 2021.

### Example 3: Confirmation of physiological characteristics of strains

Regarding the strains deposited above, physiological characteristics thereof were confirmed.

Specifically, among the physiological characteristics, carbon source availability was assessed using the API 50 CHL kit (API 50 CHL; manufacturer: BioMerieux, USA) or the API 20A kit (API 20A, BioMerieux's, USA) through a sugar fermentation test. The results are shown in Tables 3 (*Lactobacillus rhamnosus* NK210), 4 (*Bifidobacterium longum* NK219), and 5 (*Lactococcus lactis* NK209) below. In Table 3-5 below, "+" indicates a positive carbon source availability, while "-" indicates a negative carbon source availability.

**[Table 3]**

| **Carbon source** | **NK210** | **Carbon source** | **NK210** |
|---|---|---|---|
| CONTROL | - | Esculin | + |
| Glycerol | - | Salicin | + |
| Erythritol | - | Cellobiose | + |
| D-Arabinose | - | Maltose | + |
| L-Arabinose | - | Lactose | + |
| D-Ribose | - | Melibiose | - |
| D-Xylose | - | Sucrose | - |
| L-Xylose | - | Trehalose | + |
| D-Adonitol | - | Inulin | - |
| Methyl-β-D-xylopyranoside | - | Melezitose | + |
| D-Galactose | + | Raffinose | - |
| D-Glucose | + | Starch | - |
| D-Fructose | + | Glycogen | - |
| D-Mannose | + | Xylitol | - |
| L-Sorbose | - | Gentiobiose | + |
| Rhamnose | - | D-Turanose | - |
| Dulcitol | + | D-Lyxose | - |
| Inositol | - | D-Tagatose | + |
| Mannitol | + | D-Fucose | - |
| Sorbitol | + | L-Fucose | + |
| α-Methyl-D-mannoside | - | D-Arabitol | - |
| α-Methyl-D-glucoside | - | L-Arabitol | - |
| N-Acetyl-glucosamine | + | Gluconate | + |
| Amygdalin | + | 2-Keto-gluconate | - |
| Arbutin | + | 5-Keto-gluconate | - |

**[Table 4]**

| Carbon source | Reaction/Enzyme | NK219 |
|---|---|---|
| L-Tryptophan | indole formation | - |
| Urea | urease | - |
| D-Glucose | acidification(glucose) | + |
| D-mannitol | acidification(mannitol) | - |
| D-Lactose | acidification(lactose) | + |
| D-Saccharose | acidification(saccharose) | + |
| D-maltose | acidification(maltose) | + |
| salicin | acidification(salicin) | + |
| D-xylose | acidification(xylose) | + |
| L-arabinose | acidification(arabinose) | + |
| gelatin | hydrolysis(protease) | + |
| Esculin Ferric Citrate | hydrolysis(β-glucosidase) | - |
| glycerol | acidification(glycerol) | - |
| D-cellobiose | acidification(cellobiose) | - |
| D-mannose | acidification(mannose) | + |
| D-Melezitose | acidification(melezitose) | - |
| D-raffinose | acidification(raffinose) | + |
| D-Sorbitol | acidification(sorbitol) | - |
| L-rhamnose | acidification(rhamnose) | - |
| D-Trehalose | acidification(trehalose) | + |
| | Catalase | - |
| | Spores | - |
| | Gram reaction | - |
| | Morphology | - |

**[Table 5]**

| **Carbon source** | **NK209** | **Carbon source** | **NK209** |
|---|---|---|---|
| CONTROL | - | Esculin | + |
| Glycerol | - | Salicin | + |
| Erythritol | - | Cellobiose | + |
| D-Arabinose | - | Maltose | + |
| L-Arabinose | - | Lactose | - |
| D-Ribose | + | Melibiose | + |
| D-Xylose | - | Sucrose | + |
| L-Xylose | - | Trehalose | + |
| D-Adonitol | - | Inulin | - |
| Methyl-β-D-xylopyranoside | - | Melezitose | - |
| D-Galactose | + | Raffinose | + |
| D-Glucose | + | Starch | + |
| D-Fructose | + | Glycogen | - |
| D-Mannose | + | Xylitol | - |
| L-Sorbose | - | Gentiobiose | + |
| Rhamnose | - | D-Turanose | - |
| Dulcitol | - | D-Lyxose | - |
| Inositol | - | D-Tagatose | - |
| Mannitol | + | D-Fucose | - |
| Sorbitol | - | L-Fucose | - |
| α-Methyl-D-mannoside | - | D-Arabitol | - |
| α-Methyl-D-glucoside | - | L-Arabitol | - |
| N-Acetyl-glucosamine | + | Gluconate | - |
| Amygdalin | + | 2-Keto-gluconate | - |
| Arbutin | + | 5-Keto-gluconate | - |

### Example 4: Production of dead bacteria and lysates

### Example 4-1 Production of dead bacteria

*Lactobacillus rhamnosus* NK210 KCCM13049P, *Bifidobacterium longum* NK219 KCCM13050P or *Lactococcus lactis* NK209 KCCM13048P were subjected to heat treatment three times at 90°C for 10 minutes under heat treatment conditions, thereby producing dead bacteria.

### Example 4-2 Production of lysates (solubles and insolubles)

Live bacteria obtained by centrifuging *Lactobacillus rhamnosus* NK210 KCCM13049P, *Bifidobacterium longum* NK219 KCCM13050P, or *Lactococcus lactis* NK209 KCCM13048P were suspended in sterilized distillation to 1×10⁶ CFU/mL, followed by sonication at 4°C (1 minute treatment followed by 1 minute rest, repeated five times) and centrifugation (10,000 g, 4°C, 15 minutes) to obtain supernatant (soluble matter) and precipitate (insoluble matter). The obtained products were freeze-dried and used.

### Example 5. Confirmation of activity on dead bacteria and lysates

The anti-inflammatory activity in macrophages was confirmed as in Example 2 using the dead bacteria and lysates produced in Example 4.

As a result, it was confirmed in both dead bacteria produced through heat treatment (90°C, 10 minutes, 3 times) and lysates disrupted by sonication (1 minute treatment followed by 1 minute rest, repeated five times) that the inducing activity of IL-10 expression compared to TNF-α expression was similar to that when treating live bacteria.

### [Mode for Carrying out the Invention]

### Test Example

The measurement method in the following test was performed under the standards of the test method below.

### (1) Measurement of myeloperoxidase (MPO) activity

To 100 mg of colon tissue, 200 µl of 10 mM potassium phosphate buffer (pH 7.0) containing 0.5% hexadecyl trimethyl ammonium bromide was added and homogenized. Then, the supernatant was obtained by centrifugation for 10 minutes at 4°C and 10,000 g. A volume of 50 µl of the supernatant was added to 0.95 mℓ of reaction solution (containing 1.6 mM tetramethyl benzidine and 0.1 mM H₂O₂), reacted at 37°C, and absorbance was monitored over time at 650 nm.

### (2) Measurement of interferon (IFN)-γ, TNF-α, and IL-10 indicators

Hippocampus, colon, and spleen tissues were homogenized by adding 1 ml of RIPA buffer (Gibco) containing protease inhibitor cocktail and centrifuged at 4°C, 13000 rpm for 15 min to obtain supernatant. Indicators of this supernatant were measured using an ELISA kit (Ebioscience).

### (3) Memory experiment

### 1) Y-maze task experiment method:

The Y-shaped maze measurement device is shaped like an alphabetical Y with three arms extending from it, each arm being 25 cm long, 14 cm high, 5 cm wide, and positioned at the same angle. At the end of one arm of the Y-shaped maze, the animal was placed with its head facing out and allowed to freely navigate the arm for 8 minutes. The animal's movements were recorded, and reported as arm entries when the animal entered the arm with its hind paw, and the animal's movement was represented by an alternation, which was defined as one alternation when the animal passed through the three arms in succession. The amount of spontaneous alternation behavior was expressed as a percentage between the actual number of alternations and the maximum possible number of alternations (i.e., the total number of alternations minus 2).

### 2) Object recognition test:

The experiment was conducted in a box (40 × 40 × 40 cm) made so that the outside could not be seen from the inside. Two objects (A, A') of the same shape and size were fixedly disposed in a box and the mouse was relocated to start from the center of the box. Then, the number of times the mouse made contact with the two objects was recorded for 10 minutes. After 24 hours, one of the two objects was replaced with a new object (A, B). Then, one original object and one new object were prepared, and the animal's exploration time was measured.

### (4) qPCR (Quantitative real time polymerase chain reaction)

mRNA was isolated from spleen tissue using the RNA Isolation Kit: RNeasy Mini Kit (QIAGEN), converted to cDNA, and qPCR was performed to measure the expression rates of Tbet, Foxp3, and glyceraldehyde 3-phosphate dehydrogenase (GAPDH). The reaction product was first treated at 95°C for 30 seconds, followed by 38 cycles of 5 seconds at 95°C and 30 seconds at 72°C for analysis.

### Primers for qPCR

**[Table 6]**

| | | |
|---|---|---|
| Foxp3 | Forward | 5'-AGAAGCTGGGAGCTATGCAG-3' (SEQ ID NO: 6) |
| | Reverse | 5'-GCTACGATGCAGCAAGCGC-3' (SEQ ID NO: 7) |
| Tbet | Forward | 5'-TGCCCGAACTACAGTCACGAAC-3' (SEQ ID NO: 8) |
| | Reverse | 5'-AGTGACCTCGCCTGGTGAAATG-3' (SEQ ID NO: 9) |
| GAPDH | Forward | 5'-TGCAGTGGCAAAGTGGAGAT-3' (SEQ ID NO: 10) |
| | Reverse | 5'-TTTGCCGTGAGTGGAGTCATA-3' (SEQ ID NO: 11) |

### (5) Gut microbiota analysis

First, DNA from feces was isolated using the QIAamp DNA stool mini kit (Qiagen, Germany), pyrosequencing was performed by amplifying barcoded primers (V4 region of the bacterial 16S rRNA gene) to generate amplicons, and 16S rDNA was analyzed by Illumina iSeq 100 (San Diego, CA) to analyze occupancy.

### Example 6: Efficacy analysis of strain administration in animals with LPS-induced cognitive impairment

C57BL/6 male mice (5 weeks old, 19-21 g) were divided (6 mice in each group) and acclimatized to the laboratory for 1 week. To induce intestinal and brain diseases, mice were intraperitoneally administered *Escherichia coli*-derived LPS (10 ug/kg) once daily for 5 days. From the next day, each mouse was administered once a day for 5 days with lactic acid bacteria at a concentration of 1×10⁹ CFU per mouse (NK210 alone: 1×10⁹ CFU/mouse, NK219 alone: 1×10⁹ CFU/mouse, combined (210+219): 210, 8×10⁸ CFU/mouse and 219, 2×10⁸ CFU/mouse), and sulfasalazine of 50 mg/kg (mouse). Experimental animals in the normal group were orally administered with a physiological saline solution, which was used to suspend lactic acid bacteria. The day following the conclusion of sample administration, the experimental animals were sacrificed, and samples from the brain hippocampus, colon, and spleen were collected. In the Hippocampus, inflammatory indicators, such as IFN-γ, TNF-a, and IL-10, were measured using ELISA. In the colon, intestinal length and inflammatory indicators, such as IFN-γ, TNF-a, and IL-10, were measured. In the spleen, Th1 cells and Treg cells were measured by FACS (Fluorescence-Activated Cell Sorting).

### (1) Confirmation of colitis improvement effect

**[Table 7]**

| Parameter s of colon | Administration | | | | | |
|---|---|---|---|---|---|---|
| | NC | LPS | LPS+ NK 210 | LPS+ NK 219 | LPS+ Mx(4:1) (NK210+ NK219) | LPS+ Sulfasal azine |
| Colon (cm) | 5.2 | 4.7 | 5.0 | 4.8 | 5.1 | 5.0 |
| MPO activity (µUnit/mg) | 4.15 | 7.24 | 4.55 | 4.30 | 4.89 | 4.75 |
| Interferon (IFN)-γ (pg/mg) | 34.49 | 55.05 | 44.24 | 44.72 | 39.24 | 45.4 |
| TNF-α (pg/mg) | 16.59 | 24.82 | 20.76 | 18.59 | 19.68 | 20.1 |
| IL-10 (pg/mg) | 19.14 | 13.55 | 21.10 | 19.56 | 22.65 | 15.91 |
| IFN-γ/IL-10 | 1.86 | 4.12 | 2.50 | 2.35 | 1.80 | 2.85 |
| TNF-a/IL-10 | 1.02 | 1.53 | 0.98 | 0.96 | 1.01 | 1.26 |

As shown in Table 7 above, the effect of improving colitis by administration of single strains and combined strains was confirmed through changes in intestinal length, expression of inflammatory factors, and MPO activity, and the like. Specifically, the decrease in intestinal length was improved, MPO activity was reduced, the expression levels of interferon-gamma and TNF-α were decreased, and the expression level of IL-10, an anti-inflammatory cytokine, was increased. In other words, the symptoms of colitis were significantly improved by inhibiting the expression of IFN-γ and TNF-α increased by endotoxin and increasing the expression of IL-10 decreased by endotoxin, particularly increasing the expression of IL-10 compared to IFN-γ and IL-10 compared to TNF-α. It was conformed that these actions acted synergistically in the combined strains, and in particular, noteworthy was the optimal outcome observed when administering NK210 and NK219 at a mixing ratio of 4:1.

### (2) Confirmation of improvement effect in spleen

**[Table 8]**

| Parameters of spleen | Administration | | | | | |
|---|---|---|---|---|---|---|
| | NC | LPS | LPS+ NK 210 | LPS+ NK 219 | LPS+ Mx (NK210+ NK219) | LPS+ Sulfasalazine |
| Phagocytosis (%) | 37.44 | 56.63 | 36.01 | 34.55 | 36.74 | 39.59 |
| NK cell cytoxicity (%) | 6.22 | 9.73 | 7.78 | 6.93 | 7.05 | 7.92 |
| Interferon (IPN)-γ (pg/mg) | 10.10 | 15.02 | 11.92 | 11.29 | 12.18 | 13.55 |
| TNF-α (pg/mg) | 3.81 | 4.80 | 4.00 | 4.00 | 4.06 | 4.51 |
| IL-10 (pg/mg) | 10.15 | 8.23 | 10.49 | 8.71 | 9.98 | 9.78 |
| IFN-γ/IL-10 | 1.02 | 1.85 | 1.15 | 1.32 | 1.33 | 1.39 |
| TNF-a/IL-10 | 0.39 | 0.58 | 0.38 | 0.47 | 0.41 | 0.13 |
| Tbet (fold change) | 1.12 | 2.94 | 1.58 | 1.27 | 1.41 | 1.82 |
| Foxp3 (fold change) | 1.28 | 0.80 | 1.07 | 1.32 | 1.34 | 1.02 |
| Tbet/Foxp3 | 0.91 | 3.77 | 1.55 | 1.05 | 1.12 | 1.78 |

As shown in Table 8 above, it was confirmed that the phagocytic ability of macrophages, and the apoptosis effect of NK cells, which are natural immune cells, were improved. In addition, the expression of IFN-γ and TNF-α increased by endotoxin was inhibited and the expression of IL-10 decreased by endotoxin was increased, and in particular, the expression of IL-10 compared to IFN-γ and IL-10 compared to TNF-α was also increased (i.e., decrease in the ratios of IFN-γ/IL-10 and TNF-α/IL-10, same as below). This action in the spleen, which is systemically involved in the inflammatory response, demonstrates the excellent inflammation-improving effect of the strains according to the present disclosure. Moreover, this action was shown to be synergistic not only in single strains but also in combined strains, showing excellent effects. In particular, noteworthy was the optimal outcome observed when administering NK210 and NK219 at a mixing ratio of 4:1.

### (3) Confirmation of cognitive improvement effect

**[Table 9]**

| Parameter | Administration | | | | | |
|---|---|---|---|---|---|---|
| | NC | LPS | LPS+NK210 | LPS+ NK219 | LPS+ Mx (NK210+ NK219) | LPS+ Sulfasalazine |
| Spontaneous alteration (%) (in Y-maze task) | 75.85 | 61.37 | 69.36 | 71.06 | 75.45 | 69.21 |
| Exoporation (%) (in NOR task) | 79.18 | 69.95 | 76.00 | 76.97 | 81.07 | 72.02 |
| Interferon (IFN)-γ (pg/mg) | 24.04 | 31.20 | 23.04 | 24.09 | 26.95 | 27.35 |
| TNF-α (pg/mg) | 11.07 | 12.61 | 10.44 | 10.72 | 10.68 | 10.98 |
| IL-10 (pg/mg) | 48.62 | 40.43 | 46.94 | 47.71 | 50.18 | 45.79 |
| IFN-γ/IL-10 | 0.50 | 0.74 | 0.52 | 0.50 | 0.54 | 0.60 |
| TNF-a/IL-10 | 0.23 | 0.31 | 0.23 | 0.24 | 0.21 | 0.24 |

As shown in Table 9 above, administration of the strain according to the present disclosure showed excellent improvement effects in both the Y-maze task and the NOR task. In particular, this improvement effect showed a significant synergistic effect when the strains were used in combination. In addition, it was confirmed that there was an improvement effect on neuritis by improving the inflammatory indicators of the hippocampus, and in particular, lowering the levels of IFN-γ and TNF-α increased by endotoxin, increasing the expression of IL-10, and increasing the expression of IL-10 compared to IFN-γ and IL-10 compared to TNF-α.

### Example 7: Analysis of the efficacy of strain administration in immunosuppressed animals induced by anticancer drug (cyclophosphamide)

C57BL/6 male mice (5 weeks old, 19-21g) were divided (6 mice in each group) and acclimatized to the laboratory for 1 week. To induce intestinal and brain diseases, the mice were administered with cyclophosphamide (CP, 150 mg/kg, dissolved in physiological saline) for 1 day, rested for 1 day, and then administered intraperitoneally the next day. Meanwhile, the normal group was administered intraperitoneally with 0.1 ml of physiological saline. From the next day after the final administration of the anticancer drug, the mice were orally administered once a day for 5 days with lactic acid bacteria as a test sample suspended in physiological saline solution at a concentration of 1×10⁹ CFU per mouse (NK210 alone: 1×10⁹ CFU/mouse, NK219 alone: 1×10⁹ CFU/mouse, combined (210+219): 210, 8×10⁸ CFU/mouse and 219, 2×10⁸ CFU/mouse), and sulfasalazine of 50 mg/kg (mouse). The day following the conclusion of sample administration, the experimental animals were sacrificed, and samples from the brain hippocampus, colon, and spleen were collected. In the Hippocampus, inflammatory indicators, such as IFN-γ, TNF-α, and IL-10, were measured using ELISA. In the colon, intestinal length and inflammatory indicators, such as IFN-γ, TNF-α, and IL-10, were measured. In the spleen, Th1 cells and Treg cells were measured by FACS (Fluorescence-Activated Cell Sorting).

### (1) Confirmation of colitis improvement effect

**[Table 10]**

| Parameters of colon | Administration | | | | |
|---|---|---|---|---|---|
| | NC | CP | CP+ NK210 | CP+ NK219 | CP+ Mx (NK210+ NK219) |
| Colon (cm) | 5.30 | 4.87 | 4.98 | 4.95 | 5.15 |
| MPO activity (µUnit/mg) | 4.41 | 7.19 | 4.79 | 4.62 | 4.18 |
| Interferon (IFN)-γ (pg/mg) | 24.74 | 16.21 | 28.79 | 38.64 | 26.50 |
| TNF-α (pg/mg) | 18.79 | 12.34 | 20.60 | 26.73 | 18.75 |
| IL-10 (pg/mg) | 9.93 | 5.09 | 9.65 | 13.00 | 10.26 |
| IFN-γ/IL-10 | 2.50 | 3.83 | 3.05 | 3.32 | 2.65 |
| TNF-a/IL-10 | 1.93 | 2.70 | 2.19 | 2.12 | 1.89 |

The effect of administration in improving colitis was confirmed through changes in intestinal length, expression of inflammatory factors, and MPO activity. Specifically, the decrease in intestinal length was improved, MPO activity was decreased, and the expression of IFN-γ, TNF-α, and IL-10, which were suppressed by anticancer drugs, all increased. In particular, colitis was improved by increasing the expression of IL-10 compared to IFN-γ and IL-10 compared to TNF-α. It was conformed that these actions acted synergistically in the combined strains, and in particular, noteworthy was the optimal outcome observed when administering NK210 and NK219 at a mixing ratio of 4:1.

### (2) Confirmation of improvement effect in spleen

**[Table 11]**

| Parameters of spleen | Administration | | | | |
|---|---|---|---|---|---|
| | NC | CP | CP+ NK210 | CP+ NK219 | CP+ Mx (NK210+ NK219) |
| Phagocytosis (%) | 34.20 | 17.14 | 58.65 | 50.36 | 49.95 |
| NK cell cytoxicity (%) | 8.08 | 6.60 | 8.26 | 8.90 | 8.23 |
| Interferon (IFN)-γ (pg/mg) | 12.60 | 3.26 | 7.39 | 5.56 | 6.41 |
| TNF-α (pg/mg) | 4.01 | 1.86 | 3.20 | 2.45 | 3.07 |
| IL-10 (pg/mg) | 8.07 | 1.43 | 3.31 | 2.90 | 3.54 |
| IFN-γ/IL-10 | 1.58 | 2.83 | 2.31 | 2.11 | 1.85 |
| TNF-a/IL-10 | 0.50 | 1.67 | 1.04 | 0.87 | 0.90 |
| Tbet (fold change) | 1.08 | 0.68 | 0.75 | 0.95 | 0.95 |
| Foxp3 (fold change) | 0.84 | 0.25 | 0.37 | 0.36 | 0.53 |
| Tbet/Foxp3 | 1.35 | 2.66 | 2.05 | 2.49 | 1.89 |

As shown in Table 11 above, it was confirmed that the phagocytic ability of macrophages, and the apoptosis effect of NK cells, which are natural immune cells, were improved. The expression of IFN-γ, TNF-α, and IL-10, which were suppressed by anticancer drugs, all increased. In particular, excellent therapeutic effect was shown by increasing the expression of IL-10 compared to IFN-γ and IL-10 compared to TNF-α. Moreover, this action was shown to be synergistic not only in single strains but also in combined strains, showing excellent effects. In particular, noteworthy was the optimal outcome observed when administering NK210 and NK219 at a mixing ratio of 4:1.

### (3) Confirmation of cognitive improvement effect

**[Table 121**

| Parameter | Administration | | | | |
|---|---|---|---|---|---|
| | NC | CP | CP+ NK210 | CP+ NK219 | CP+ Mx (NK210+ NK219) |
| Spontaneous alteration (%) (in Y-maze task) | 78.95 | 58.95 | 70.94 | 71.26 | 71.39 |
| Exoporation (%) (in NOR task) | 80.55 | 62.40 | 78.71 | 78.34 | 80.48 |
| Interferon (IFN)-γ (pg/mg) | 27.21 | 23.15 | 25.30 | 23.48 | 27.84 |
| TNF-α(pg/mg) | 11.55 | 10.60 | 10.44 | 10.91 | 12.00 |
| IL-10(pg/mg) | 52.19 | 40.07 | 43.29 | 53.74 | 59.15 |
| IFN-γ/IL-10 | 0.52 | 0.58 | 0.59 | 0.44 | 0.47 |
| TNF-a/IL-10 | 0.22 | 0.27 | 0.24 | 0.20 | 0.20 |

As shown in Table 12 above, administration of the strain according to the present disclosure showed excellent improvement effects in both the Y-maze task and the NOR task. In particular, this improvement effect showed a significant synergistic effect when the strains were used in combination. In addition, the expression of IFN-γ, TNF-α, and IL-10, which were suppressed by anticancer drugs, all increased, and in particular, the expression of IL-10 compared to IFN-γ and IL-10 compared to TNF-α was improved, resulting in a therapeutic effect (showed an immunoregulation effect).

### Example 8: Efficacy analysis by strain treatment in animals with antibiotic-induced gut microbiota imbalance

C57BL/6 male mice (5 weeks old, 19-21g) were divided (6 mice in each group) and acclimatized to the laboratory for 1 week. To induce intestinal and brain diseases, the mice were orally administered with ampicillin (AP, 100 mg/kg, dissolved in physiological saline) for 3 days. Meanwhile, the normal group was orally administered with 0.1 ml of physiological saline. From the next day after the final administration of the antibiotic, the mice were orally administered once a day for 5 days with lactic acid bacteria as a test sample suspended in physiological saline solution at a concentration of 1×10⁹ CFU per mouse (NK210 alone: 1×10⁹ CFU/mouse, NK219 alone: 1×10⁹ CFU/mouse, combined (210+219): 210, 8×10⁸ CFU/mouse and 219, 2×10⁸ CFU/mouse), and sulfasalazine of 50 mg/kg (mouse). The day following the conclusion of sample administration, the experimental animals were sacrificed, and samples from the brain hippocampus, colon, and spleen were collected. In the Hippocampus, inflammatory indicators, such as IFN-γ, TNF-α, and IL-10, were measured using ELISA. In the colon, intestinal length and inflammatory indicators, such as IFN-γ, TNF-α, and IL-10, were measured. In the spleen, Th1 cells and Treg cells were measured by FACS (Fluorescence-Activated Cell Sorting), and in feces, endotoxin (LPS) and gut microbial communities were measured.

### (1) Confirmation of colitis improvement effect

**[Table 13]**

| Parameters of colon | Administration | | | | |
|---|---|---|---|---|---|
| | NC | AP | AP+NK210 | AP+NK219 | AP+Mx (NK210+ NK219) |
| Colon (cm) | 5.12 | 4.53 | 5.28 | 5.45 | 5.48 |
| MPO activity (µUnit/mg) | 10.96 | 20.14 | 11.60 | 14.76 | 11.05 |
| Interferon (IFN)-γ (pg/mg) | 6.52 | 9.87 | 5.94 | 5.84 | 6.42 |
| TNF-α(pg/mg) | 23.08 | 33.10 | 22.86 | 28.27 | 19.62 |
| IL-10(pg/mg) | 19.91 | 13.05 | 21.31 | 26.75 | 20.40 |
| IFN-γ/IL-10 | 0.32 | 0.55 | 0.31 | 0.26 | 0.36 |
| TNF-a/IL-10 | 1.14 | 2.64 | 1.43 | 1.16 | 1.05 |

The effect of administration in improving colitis was confirmed through changes in intestinal length, expression of inflammatory factors, and MPO activity. Specifically, the decrease in intestinal length was improved, MPO activity was reduced, the expression levels of interferon-gamma and TNF-α were decreased, and the expression level of IL-10, an anti-inflammatory cytokine, was increased. In other words, the symptoms of colitis were significantly improved by inhibiting the expression of IFN-γ and TNF-α increased by endotoxin and increasing the expression of IL-10 decreased by endotoxin, especially increasing the expression of IL-10 compared to IFN-γ and IL-10 compared to TNF-α. It was conformed that these actions acted synergistically in the combined strains, and in particular, noteworthy was the optimal outcome observed when administering NK210 and NK219 at a mixing ratio of 4:1.

### (2) Confirmation of improvement effect in spleen

**[Table 14]**

| Parameters of spleen | Administration | | | | |
|---|---|---|---|---|---|
| | NC | AP | AP+ NK210 | AP+ NK219 | AP+ Mx (NK210+ NK219) |
| Phagocytosis (%) | 42.46 | 21.26 | 74.02 | 69.91 | 72.79 |
| NK cell cytoxicity (%) | 9.04 | 5.14 | 7.34 | 6.47 | 9.46 |
| Interferon (IFN)-γ (pg/mg) | 7.82 | 7.91 | 7.03 | 9.12 | 10.29 |
| TNF-α (pg/mg) | 3.41 | 3.96 | 3.57 | 4.32 | 5.01 |
| IL-10 (pg/mg) | 9.56 | 7.78 | 8.25 | 11.35 | 13.43 |
| IFN-γ/IL-10 | 0.82 | 1.03 | 0.87 | 0.81 | 0.77 |
| TNF-a/IL-10 | 0.36 | 0.52 | 0.45 | 0.40 | 0.41 |
| Tbet (fold change) | 0.86 | 1.42 | 1.07 | 1.03 | 0.98 |
| Foxp3 (fold | 1.27 | 0.62 | 0.69 | 1.66 | 1.39 |
| change) | | | | | |
| Tbet/Foxp3 | 0.76 | 2.39 | 1.66 | 0.74 | 0.87 |

As shown in Table 14 above, it was confirmed that the phagocytic ability of macrophages, and the apoptosis effect of NK cells, which are natural immune cells, were improved. In addition, the expression of IFN-γ and TNF-α increased by endotoxin was inhibited, while the expression of IL-10 decreased by endotoxin was increased, particularly increasing the expression of IL-10 compared to IFN-γ and IL-10 compared to TNF-α. This action in the spleen, which is systemically involved in the inflammatory response, demonstrates the excellent inflammation-improving effect of the strain according to the present disclosure. Moreover, this action was shown to be synergistic not only in single strains but also in combined strains, showing excellent effects. In particular, noteworthy was the optimal outcome observed when administering NK210 and NK219 at a mixing ratio of 4:1.

### (3) Confirmation of cognitive improvement effect

**[Table 15]**

| Parameter | Administration | | | | |
|---|---|---|---|---|---|
| | NC | AP | AP+ NK210 | AP+ NK219 | AP+ Mx (NK210+ NK219) |
| Colon (cm) | 5.12 | 4.53 | 5.28 | 5.45 | 5.48 |
| Spontaneous alteration (%) (in Y-maze task) | 72.97 | 57.36 | 73.01 | 70.54 | 70.90 |
| Exoporation (%) (in NOR task) | 80.26 | 71.32 | 76.25 | 83.21 | 82.45 |
| Interferon (IFN)-γ (pg/mg) | 25.45 | 29.39 | 23.18 | 24.33 | 24.79 |
| TNF-α (pg/mg) | 12.41 | 16.51 | 13.37 | 12.43 | 14.09 |
| IL-10 (pg/mg) | 47.34 | 16.52 | 48.89 | 50.29 | 46.29 |
| IFN-γ/IL-10 | 0.54 | 1.78 | 0.47 | 0.48 | 0.54 |
| TNF-a/IL-10 | 0.27 | 0.41 | 0.26 | 0.28 | 0.31 |

As shown in Table 15 above, administration of the strain according to the present disclosure showed excellent improvement effects in both the Y-maze task and the NOR task. In particular, this improvement effect showed a significant synergistic effect when the strains were used in combination. In addition, it was confirmed that there was an improvement effect on neuritis by improving the inflammatory indicators of the hippocampus, and in particular, lowering the levels of IFN-γ and TNF-α increased by endotoxin, increasing the expression of IL-10, and increasing the expression of IL-10 compared to IFN-γ and IL-10 compared to TNF-α.

### (4) Improvement of gut microbiota imbalance

The results of improving gut microbiota imbalance are shown in FIG. 1. As illustrated in FIG. 1, it was confirmed that the imbalance in the beta diversity, Proteobacteria, and Bacteroidetes of gut microbiota reduced by the administration of the antibiotic ampicillin, was improved to a balanced form in the gut microbiota by administration of LR (NK210), BL (NK219), and mixture (Mx) thereof.

### Example 9: Efficacy analysis of strain administration in healthy animal

C57BL/6 male mice (5 weeks old, 19-21g) were divided (6 mice in each group) and acclimatized to the laboratory for 1 week. The mice were orally administered once a day for 5 days with lactic acid bacteria as a test sample suspended in physiological saline solution at a concentration of 1×10⁹ CFU per mouse (NK210 alone (LR) : 1×10⁹ CFU/mouse, NK219 alone (BL): 1×10⁹ CFU/mouse, combined (210+219): 210, 8×10⁸ CFU/mouse and 219, 2×10⁸ CFU/mouse). The experimental animals in the positive control group were orally administered with 50 mg/kg of sulfasalazine, a colitis treatment drug, instead of lactic acid bacteria, and the experimental animals in the normal group were orally administered with saline solution used to suspend lactic acid bacteria. The day following the conclusion of sample administration, the experimental animals were sacrificed, and samples from the brain hippocampus, colon, and spleen were collected. In the Hippocampus, inflammatory indicators, such as IFN-γ, TNF-α, and IL-10, were measured using ELISA. In the colon, intestinal length and inflammatory indicators, such as IFN-γ, TNF-α, and IL-10, were measured. In the spleen, Th1 cells and Treg cells were measured by FACS (Fluorescence-Activated Cell Sorting).

### (1) Confirmation of colitis improvement effect

**[Table 16]**

| Parameters of colon | Administration | | | |
|---|---|---|---|---|
| | NC | LR | BL | Mx |
| Colon (cm) | 5.28 | 5.12 | 5.03 | 4.97 |
| MPO activity (µUnit/mg) | 4.15 | 4.18 | 3.53 | 5.01 |
| Interferon (IFN)-γ (pg/mg) | 55.49 | 55.75 | 64.83 | 68.59 |
| TNF-α (pg/mg) | 13.78 | 13.40 | 15.73 | 14.34 |
| IL-10 (pg/mg) | 42.12 | 39.95 | 42.89 | 40.13 |
| IFN-γ/IL-10 | 1.32 | 1.40 | 1.52 | 1.73 |
| TNF-a/IL-10 | 0.33 | 0.34 | 0.37 | 0.36 |

As shown in Table 16 above, it was confirmed that the administration of single strains in healthy animals increased the expression of interferon gamma, and that the administration of combined strain increased its expression very significantly. In particular, noteworthy was the optimal outcome observed when administering NK210 and NK219 at a mixing ratio of 4:1.

### (2) Confirmation of improvement effect in spleen

**[Table 17]**

| Parameters of spleen | Administration | | | |
|---|---|---|---|---|
| | NC | LR | BL | Mx |
| Phagocytosis (%) | 37.44 | 50.03 | 64.57 | 65.45 |
| NK cell cytoxicity (%) | 6.22 | 6.65 | 8.52 | 9.31 |
| Interferon (IFN)-γ (pg/mg) | 10.10 | 13.02 | 12.56 | 12.32 |
| TNF-α (pg/mg) | 3.81 | 3.48 | 3.49 | 3.96 |
| IL-10 (pg/mg) | 8.66 | 9.45 | 9.29 | 9.68 |
| IFN-γ/IL-10 | 1.17 | 1.38 | 1.35 | 1.27 |
| TNF-a/IL-10 | 0.44 | 0.37 | 0.38 | 0.41 |
| Tbet (fold change) | 1.49 | 1.41 | 1.80 | 1.88 |
| Foxp3 (fold change) | 1.05 | 1.05 | 1.05 | 1.60 |
| Tbet/Foxp3 | 1.27 | 1.45 | 1.86 | 1.48 |

As shown in Table 17 above, it was confirmed that the phagocytic ability of macrophages, and the apoptosis effect of NK cells, which are natural immune cells, were improved. In addition, the expression of interferon gamma was increased. Moreover, this action significantly increased the expression of IFN-γ when used in combination. In particular, the expression of IL-10 compared to IFN-γ and IL-10 compared to TNF-α was improved. It was confirmed through the above results that the strains according to the present disclosure had excellent immune-enhancing efficacy.

### Example 10: Production of animals with intestinal and brain diseases induced by TNBS and confirmation of efficacy by administration of strains

C57BL/6 male mice (5 weeks old, 19-21g) were divided (6 mice in each group) and acclimatized to the laboratory for 1 week. To induce intestinal and brain diseases, mice were injected with 0.1 ml of a 5% 2,4,6-trinitrobenzenesulfonic acid (TNBS, Sigma, USA) solution diluted 1:1 in 50% ethanol, into the colon via the anus using a 1 ml volume syringe with a rounded tip, and held vertically for 30 seconds. Meanwhile, the normal group was orally administered with 0.1 ml of physiological saline. From the next day, the mice were orally administered once a day for 5 days with lactic acid bacteria as a test sample suspended in physiological saline solution at a concentration of 1×10⁹ CFU per mouse (NK210 alone (LR): 1×10⁹ CFU/mouse, NK219 alone (BL): 1×10⁹ CFU/mouse, NK209 alone (LL) : 1×10⁹ CFU/mouse, combined (MX, 210+219) : NK210, 8×10⁸ CFU/mouse and NK219, 2×10⁸ CFU/mouse, combined (MX, 209+219) : NK209, 8×10⁸ CFU/mouse and NK219, 2×10⁸ CFU/mouse, LR+BL+LL: NK210, 4×10⁸ CFU/mouse, NK219, 2×10⁸ CFU/mouse, and NK209, 4×10⁸ CFU/mouse, heat treated (H-), respectively: 1×10⁹ CFU/mouse) . The experimental animals in the positive control group were orally administered with 50 mg/kg of sulfasalazine, a colitis treatment drug, instead of lactic acid bacteria, and the experimental animals in the normal group were orally administered with saline solution used to suspend lactic acid bacteria. The day following the conclusion of sample administration, the experimental animals were sacrificed, and samples from the brain hippocampus, colon, and spleen were collected. In the Hippocampus, inflammatory indicators, such as TNF-α and IL-10, were measured using ELISA. In the colon, intestinal length and inflammatory indicators, such as TNF-α and IL-10, were measured. In the spleen, Th1 cells and Treg cells were measured by FACS (Fluorescence-Activated Cell Sorting).

### (1) Confirmation of cognitive improvement effect

**[Table 18]**

| Parameters of brain | Cognitive function | | Concentration in the hippocampus | | |
|---|---|---|---|---|---|
| | Spont. alteration (%) | Exploration (%) | IFN-γ (pg/mg) | TNF-α (pg/mg) | IL-10 (pg/mg) |
| NC | 69.31 | 78.27 | 20.73 | 9.39 | 45.51 |
| TNBS | 52.12 | 66.84 | 34.15 | 15.23 | 30.62 |
| TNBS+ LR(NK210) | 62.45 | 74.25 | 26.28 | 12.14 | 38.64 |
| TNBS+ BL(NK219) | 65.32 | 75.37 | 25.02 | 11.93 | 40.26 |
| TNBS+ LL(NK209) | 58.8 | 70.20 | 30.81 | 13.54 | 35.53 |
| TNBS+ Mx(LR+ BL=4:1) | 68.48 | 77.44 | 24.34 | 11.53 | 40.97 |
| TNBS+Mx(LL+BL=4:1) | 67.82 | 76.35 | 25.38 | 11.89 | 41.04 |
| TNBS+LR+BL+LL* | 69.14 | 77.89 | 31.25 | 10.54 | 41.53 |
| TNBS+H(heat-treated)-LR | 60.24 | 72.34 | 28.25 | 12.76 | 35.71 |
| TNBS+H-BL | 62.45 | 73.32 | 26.27 | 12.13 | 38.65 |
| TNBS+H-LL | 58..22 | 72.34 | 28.25 | 12.76 | 35.71 |
| TNBS+Sulfasal. | 59.64 | 57.69 | 30.28 | 13.45 | 35.21 |

As shown in Table 18 above, both NK210 and NK219 improved cognitive impairment caused by TNBS. In particular, the inflammatory indicators of the hippocampus, IFN-gamma and TNF-alpha, which were increased by TNBS, were inhibited, whereas IL-10 was increased. In addition, the same effect as above was observed not only for live bacteria but also for heat-treated dead bacteria. In addition, it was confirmed that the synergy effect was excellent not only when used alone, but also when used in combination with two types (NK210 and NK219), and in particular, the synergy effect was the best when used in combination with three types (NK210, NK219 and NK209).

All of the above effects were confirmed to be superior to sulfasalazine, which is currently used as a pharmaceutical.

### (2) Confirmation of improvement effect in colon

**[Table 19]**

| Parameters of colon | Colon | | | | |
|---|---|---|---|---|---|
| | length (cm) | MPO (µUnit/mg) | IFN-γ (pg/mg) | TNF-α (pg/mg) | IL-10 (pg/mg) |
| NC | 5.22 | 10.19 | 4.86 | 14.54 | 20.91 |
| TNBS | 4.22 | 25.55 | 12.51 | 32.36 | 9.56 |
| TNBS+LR(NK210) | 4.63 | 18.43 | 8.42 | 23.84 | 15.31 |
| TNBS+BL(NK219) | 4.82 | 16.24 | 7.79 | 20.57 | 17.20 |
| TNBS+LL(NK209) | 4.89 | 15.65 | 7.89 | 20.12 | 17.10 |
| TNBS+Mx(LR+BL=4:1) | 4.98 | 15.26 | 7.56 | 19.23 | 17.92 |
| TNBS+Mx(LL+BL=4:1) | 4.98 | 15.26 | 7.56 | 19.23 | 17.92 |
| TNBS+LR+BL+LL* | 5.11 | 14.94 | 7.12 | 18.54 | 17.89 |
| TNBS+H(heat-treated)-LR | 4.58 | 19.21 | 9.83 | 25.82 | 14.69 |
| TNBS+H-BL | 4.75 | 17.48 | 8.54 | 23.19 | 15.98 |
| TNBS+H-LL | 4.66 | 18.51 | 9.01 | 25.82 | 15.09 |
| TNBS+Sulfasal. | 4.52 | 19.38 | 9.89 | 26.09 | 13.87 |

As shown in Table 19 above, colitis damaged by TNBS was improved by strain treatment according to the present disclosure. In particular, the inflammatory indicators of the hippocampus, myeloperoxidase (MPO) activity, IFN-gamma, and TNF-alpha, which were increased by TNBS, were inhibited, whereas IL-10 was increased. The same effect as above was observed not only for live bacteria but also for heat-treated dead bacteria. In particular, among the combined strains, the effect of the three types of combination of NK210, NK219 and NK209 was the best, and not only each strain but also combined strains showed better results than sulfasalazine, which is currently used as a a pharmaceutical.

### <Information on deposit of lactic acid bacteria>

The present inventors have deposited a patent of *Lactobacillus rhamnosus* NK210 into the Korean Culture Center of Microorganisms, accredited depository institution (Address: Yulim Building, 45, 2ga-gil, Hongjenae, Seodaemun-gu, Seoul, Korea), and obtained an accession number of KCCM13049P on September 15, 2021.

The present inventors have deposited a patent of *Bifidobacterium longum* NK219 into the Korean Culture Center of Microorganisms, accredited depository institution (Address: Yulim Building, 45, 2ga-gil, Hongjenae, Seodaemun-gu, Seoul, Korea), and obtained an accession number of KCCM13050P on September 15, 2021.

The present inventors have deposited a patent of *Lactococcus lactis* NK209 into the Korean Culture Center of Microorganisms, accredited depository institution (Address: Yulim Building, 45, 2ga-gil, Hongjenae, Seodaemun-gu, Seoul, Korea), and obtained an accession number of KCCM13048P on September 15, 2021.

### [Accession numbers]

Name of Depository Institution: Korean Culture Center of Microorganisms (Overseas)
Accession number: KCCM13048P
Accession date: 20210915
Name of depository institution: Korean Culture Center of Microorganisms (Overseas)
Accession number: KCCM13049P
Accession date: 20210915
Name of depository institution: Korean Culture Center of Microorganisms (Overseas)
Accession number: KCCM13050P
Accession date: 20210915

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Dong-Hyun Kim
PBLBioLab,
92-24, Seonjam-Ro,
Seongbuk-Gu,
Seoul, Korea

RECEIPT IN THE CASE OF AN ORIGINAL DEPOSIT
Issued pursuant to Rule 7.1 by the
INTERNATIONAL DEPOSITARY AUTHORITY
Identified at the bottom of this page

I. IDENTIFICATION OF THE MICROORGANISM
Identification reference given by the
DEPOSITOR:
Lactococcus lactis NK209 | Accession number given by the
INTERNATIONAL DEPOSITARY
AUTHORITY:
KCCM13048P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above, which
was received by in on September. 15. 2021 (date of the original deposit).
IV. RECEIPT OF REQUEST FOR CONVERSION
The microorganism identified under I above was received by the International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY

Name : Korean Culture Center of Microorganisms | Signature(s) of person(s) having the power to
Address : Yurim B/D | represent the International Depositary
45, Hongjenae-2ga-gil | Authority or of authorized official(s):
Seodaemun-gu |
SEOUL 03641 | Date : September. 15. 2021.
Republic of Krea |

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Dong-Hyun Kim
PBLBioLab,
92-24, Seonjam-Ro,
Seongbuk-Gu,
Seoul, Korea

RECEIPT IN THE CASE OF AN ORIGINAL DEPOSIT
Issued pursuant to Rule 7.1 by the
INTERNATIONAL DEPOSITARY AUTHORITY
Identified at the bottom of this page

I. IDENTIFICATION OF THE MICROORGANISM
Identification reference given by the
DEPOSITOR:
Lactobacillus rhamnosus NK210 | Accession number given by the
INTERNATIONAL DEPOSITARY
AUTHORITY:
KCCM13049P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above, which
was received by in on September. 15. 2021 (date of the original deposit).
IV. RECEIPT OF REQUEST FOR CONVERSION
The microorganism identified under I above was received by the International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY

Name : Korean Culture Center of Microorganisms | Signature(s) of person(s) having the power to
Address : Yurim B/D | represent the International Depositary
45, Hongjenae-2ga-gil | Authority or of authorized official(s):
Seodaemun-gu |
SEOUL 03641 | Date : September. 15. 2021.
Republic of Krea |

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Dong-Hyun Kim
PBLBioLab,
92-24, Seonjam-Ro,
Seongbuk-Gu,
Seoul, Korea

RECEIPT IN THE CASE OF AN ORIGINAL DEPOSIT
Issued pursuant to Rule 7.1 by the
INTERNATIONAL DEPOSITARY AUTHORITY
Identified at the bottom of this page

I. IDENTIFICATION OF THE MICROORGANISM
Identification reference given by the
DEPOSITOR:
Bifidobacterium longum NK219 | Accession number given by the
INTERNATIONAL DEPOSITARY
AUTHORITY:
KCCM13050P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above, which
was received by in on September. 15. 2021 (date of the original deposit).
IV. RECEIPT OF REQUEST FOR CONVERSION
The microorganism identified under I above was received by the International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY

Name : Korean Culture Center of Microorganisms | Signature(s) of person(s) having the power to
Address : Yurim B/D | represent the International Depositary
45, Hongjenae-2ga-gil | Authority or of authorized official(s):
Seodaemun-gu |
SEOUL 03641 | Date : September. 15. 2021.
Republic of Krea |

### [Industrial Applicability]

The novel probiotics according to the present disclosure have high utility as a medicine or food and, in particular, can be used for preventing, treating, or relieving inflammatory diseases and cognitive dysfunction and for improving immune function, due to having an excellent antioxidant effect, inflammation-relieving effect, nerve-improving effect, cognition-improving effect, intestinal microbiota imbalance-improving effect, immune-regulating effect, etc.

## Claims

1. *Lactobacillus rhamnosus* NK210 KCCM13049P.

2. The *Lactobacillus rhamnosus* NK210 KCCM13049P of claim 1, wherein the *Lactobacillus rhamnosus* NK210 comprises 16S rDNA sequence of SEQ ID NO: 1.

3. *Bifidobacterium longum* NK219 KCCM13050P.

4. The *Bifidobacterium longum* NK219 of claim 3, wherein the *Bifidobacterium longum* NK219 comprises 16S rDNA sequence of SEQ ID NO: 2.

5. *Lactococcus lactis* NK209 KCCM13048P.

6. The *Lactococcus lactis* NK209 KCCM13048P of claim 5, wherein the *Lactococcus lactis* NK209 comprises 16S rDNA sequence of SEQ ID NO: 3.

7. A pharmaceutical composition comprising *Lactobacillus rhamnosus* NK210 KCCM13049P, *Bifidobacterium longum* NK219 KCCM13050P, *Lactococcus lactis* NK209 KCCM13048P, or any mixture thereof; and a pharmaceutically acceptable carrier.

8. A pharmaceutical composition for preventing or treating an inflammatory disease, comprising *Lactobacillus rhamnosus* NK210 KCCM13049P, *Bifidobacterium longum* NK219 KCCM13050P, *Lactococcus lactis* NK209 KCCM13048P, or any mixture thereof.

9. The pharmaceutical composition of claim 8, wherein the inflammatory disease is at least one selected from the group consisting of inflammatory bowel disease, arthritis, gout, hepatitis, asthma, obesity, keratitis, gastritis, nephritis, colitis, diabetes, tuberculosis, bronchitis, pleurisy, peritonitis, spondylitis, pancreatitis, urethritis, cystitis, vaginitis, arteriosclerosis, sepsis, burns, dermatitis, periodontitis, and gingivitis.

10. The pharmaceutical composition of claim 9, wherein the inflammatory bowel disease is at least one selected from the group consisting of ulcerative colitis, Crohn's disease, collagenous colitis, lymphocytic colitis, ischemic colitis, diversion colitis, Behcet's disease, indeterminate colitis, antibiotic-induced colitis, and anticancer drug-induced colitis.

11. A pharmaceutical composition for preventing or treating cognitive dysfunction, comprising *Lactobacillus rhamnosus* NK210 KCCM13049P, *Bifidobacterium longum* NK219 KCCM13050P, *Lactococcus lactis* NK209 KCCM13048P, or any mixture thereof.

12. The pharmaceutical composition of claim 11, wherein the cognitive dysfunction is at least one selected from the group consisting of anxiety, depression, migraine, stress, Alzheimer's disease, Huntington's disease, vascular dementia, Pick's disease, Parkinson's disease, Creutzfeldt-Jakob disease, dementia, and a combination thereof.

13. A food composition for preventing or improving an inflammatory disease, comprising *Lactobacillus rhamnosus* NK210 KCCM13049P, *Bifidobacterium longum* NK219 KCCM13050P, *Lactococcus lactis* NK209 KCCM13048P, or any mixture thereof.

14. The food composition of claim 13, wherein the inflammatory disease is at least one selected from the group consisting of inflammatory bowel disease, arthritis, gout, hepatitis, asthma, obesity, keratitis, gastritis, nephritis, colitis, diabetes, tuberculosis, bronchitis, pleurisy, peritonitis, spondylitis, pancreatitis, urethritis, cystitis, vaginitis, arteriosclerosis, sepsis, burns, dermatitis, periodontitis, and gingivitis.

15. The food composition of claim 14, wherein the inflammatory bowel disease is at least one selected from the group consisting of ulcerative colitis, Crohn's disease, collagenous colitis, lymphocytic colitis, ischemic colitis, diversion colitis, Behcet's disease, indeterminate colitis, antibiotic-induced colitis, and anticancer drug-induced colitis.

16. A food composition for preventing or improving cognitive dysfunction, comprising *Lactobacillus rhamnosus* NK210 KCCM13049P, *Bifidobacterium longum* NK219 KCCM13050P, *Lactococcus lactis* NK209 KCCM13048P, or any mixture thereof.

17. The food composition of claim 16, wherein the cognitive dysfunction is at least one selected from the group consisting of anxiety, depression, migraine, stress, Alzheimer's disease, Huntington's disease, vascular dementia, Pick's disease, Parkinson's disease, Creutzfeldt-Jakob disease, dementia, and a combination thereof.

18. A composition for immunoregulation, comprising *Lactobacillus rhamnosus* NK210 KCCM13049P, *Bifidobacterium longum* NK219 KCCM13050P, *Lactococcus lactis* NK209 KCCM13048P, or any mixture thereof.

19. A composition for improving immune function, comprising *Lactobacillus rhamnosus* NK210 KCCM13049P, *Bifidobacterium longum* NK219 KCCM13050P, *Lactococcus lactis* NK209 KCCM13048P, or any mixture thereof.

20. A method for preventing or treating an inflammatory disease, comprising administering to a subject *Lactobacillus rhamnosus* NK210 KCCM13049P, *Bifidobacterium longum* NK219 KCCM13050P, *Lactococcus lactis* NK209 KCCM13048P, or any mixture thereof.

21. The method of claim 20, wherein the inflammatory disease is at least one selected from the group consisting of inflammatory bowel disease, arthritis, gout, hepatitis, asthma, obesity, keratitis, gastritis, nephritis, colitis, diabetes, tuberculosis, bronchitis, pleurisy, peritonitis, spondylitis, pancreatitis, urethritis, cystitis, vaginitis, arteriosclerosis, sepsis, burns, dermatitis, periodontitis, and gingivitis.

22. The method of claim 21, wherein the inflammatory bowel disease is at least one selected from the group consisting of ulcerative colitis, Crohn's disease, collagenous colitis, lymphocytic colitis, ischemic colitis, diversion colitis, Behcet's disease, indeterminate colitis, antibiotic-induced colitis, and anticancer drug-induced colitis.

23. A method for preventing or treating cognitive dysfunction, comprising administering to a subject *Lactobacillus rhamnosus* NK210 KCCM13049P, *Bifidobacterium longum* NK219 KCCM13050P, *Lactococcus lactis* NK209 KCCM13048P, or any mixture thereof.

24. The method of claim 23, wherein the cognitive dysfunction is at least one selected from the group consisting of anxiety, depression, migraine, stress, Alzheimer's disease, Huntington's disease, vascular dementia, Pick's disease, Parkinson's disease, Creutzfeldt-Jakob disease, dementia, and a combination thereof.

25. A method for immunoregulation, comprising administering to a subject *Lactobacillus rhamnosus* NK210 KCCM13049P, *Bifidobacterium longum* NK219 KCCM13050P, *Lactococcus lactis* NK209 KCCM13048P, or any mixture thereof.

26. A method for improving immune function, comprising administering to a subject *Lactobacillus rhamnosus* NK210 KCCM13049P, *Bifidobacterium longum* NK219 KCCM13050P, *Lactococcus lactis* NK209 KCCM13048P, or any mixture thereof.

27. Use of *Lactobacillus rhamnosus* NK210 KCCM13049P, *Bifidobacterium longum* NK219 KCCM13050P, *Lactococcus lactis* NK209 KCCM13048P, or any mixture thereof, in the manufacture of a medicament for the treatment of an inflammatory disease.

28. The use of claim 27, wherein the inflammatory disease is at least one selected from the group consisting of inflammatory bowel disease, arthritis, gout, hepatitis, asthma, obesity, keratitis, gastritis, nephritis, colitis, diabetes, tuberculosis, bronchitis, pleurisy, peritonitis, spondylitis, pancreatitis, urethritis, cystitis, vaginitis, arteriosclerosis, sepsis, burns, dermatitis, periodontitis, and gingivitis.

29. The use of claim 28, wherein the inflammatory bowel disease is at least one selected from the group consisting of ulcerative colitis, Crohn's disease, collagenous colitis, lymphocytic colitis, ischemic colitis, diversion colitis, Behcet's disease, indeterminate colitis, antibiotic-induced colitis, and anticancer drug-induced colitis.

30. Use of *Lactobacillus rhamnosus* NK210 KCCM13049P, *Bifidobacterium longum* NK219 KCCM13050P, *Lactococcus lactis* NK209 KCCM13048P, or any mixture thereof, in the manufacture of a medicament for the treatment of cognitive dysfunction.

31. The use of claim 30, wherein the cognitive dysfunction is at least one selected from the group consisting of anxiety, depression, migraine, stress, Alzheimer's disease, Huntington's disease, vascular dementia, Pick's disease, Parkinson's disease, Creutzfeldt-Jakob disease, dementia, and a combination thereof.

32. Use of *Lactobacillus rhamnosus* NK210 KCCM13049P, *Bifidobacterium longum* NK219 KCCM13050P, *Lactococcus lactis* NK209 KCCM13048P, or any mixture thereof, in the manufacture of a medicament for use in immunoregulation.

33. Use of *Lactobacillus rhamnosus* NK210 KCCM13049P, *Bifidobacterium longum* NK219 KCCM13050P, *Lactococcus lactis* NK209 KCCM13048P, or any mixture thereof, in the manufacture of a medicament for use in improving immune function.
